(19) Europäisches Patentamt
European Patent Office
Office européen des brevets

(11) EP 4 302 687 A1

(12) EUROPEAN PATENT APPLICATION

(43) Date of publication:
10.01.2024 Bulletin 2024/02

(51) International Patent Classification (IPC):
$A61B\ 5/022^{(2006.01)}$   $A61B\ 5/00^{(2006.01)}$

(21) Application number: 22189833.1

(22) Date of filing: 11.08.2022

(52) Cooperative Patent Classification (CPC):
A61B 5/022; A61B 5/02225; A61B 5/7235;
A61B 5/7242

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 08.07.2022 US 202263359240 P

(71) Applicant: Koninklijke Philips N.V.
5656 AG Eindhoven (NL)

(72) Inventors:
• STOLZE, Benjamin
Eindhoven (NL)

• REGH, Stephan Guido Maria
Eindhoven (NL)
• PFEIFFER, Ulrich
5656AG Eindhoven (NL)
• YAVARIMANESH, Mohammad
5656AG Eindhoven (NL)
• GAO, Mingwu
Eindhoven (NL)
• SARRICOLEA VALENCIANO, Pablo
Eindhoven (NL)
• HAIDAR, Samer
Eindhoven (NL)

(74) Representative: Philips Intellectual Property &
Standards
High Tech Campus 52
5656 AG Eindhoven (NL)

(54) APPARATUS FOR DETERMINING BLOOD PRESSURE OF A SUBJECT

(57) An apparatus for determining blood pressure of a subject is presented. The apparatus comprises a pressure signal providing unit configured to provide a measured pressure signal (TP) of a subject over a time, wherein the pressure signal is indicative of blood pulsations and comprises a plurality of pressure pulses. The apparatus further comprises a processor that is configured to determine, based on the plurality of pressure pulses, a blood pressure determination curve (TPW_M-curve) indicative for a dependence of the pressure pulses on the applied pressure, to apply a transformation to the blood pressure determination curve and to determine the blood pressure based on a result of the transformation. This allows to determine the blood pressure more accurately.

FIG. 11

**Description**

FIELD OF THE INVENTION

The invention relates to an apparatus, a method and a computer program for determining blood pressure of a subject.

BACKGROUND OF THE INVENTION

EP 3 430 992 A1 discloses a blood pressure measuring system configured to surround a subject's body part, wherein the blood pressure measuring system comprises pressurization means for applying pressure to the body part and a kinking-proof shell. The kinking-proof shell is arranged to be located between the pressurization means and the body part, when the blood pressure measuring system surrounds the body part. The blood pressure measuring system allows measuring the blood pressure by continuously raising clamping pressure and stopping the rise above systolic blood pressure.

SUMMARY OF THE INVENTION

It is an object of the present invention to provide an improved apparatus, method and computer program for determining blood pressure which allow, for instance, to determine the blood pressure more accurately.
In a first aspect of the invention an apparatus for determining blood pressure of a subject is presented, wherein the apparatus comprises:

- a pressure signal providing unit configured to provide a measured pressure signal of a subject over a time, wherein the pressure signal has been measured by using a measurement device comprising a) a shell configured to encase a part of the subject, through which blood flows, b) a pressure applicator configured to apply, from outside the shell, pressure to the shell and thereby to the encased part of the subject, and c) a pressure sensor configured to measure the pressure signal on the skin of the encased part of the subject, wherein the pressure applicator increases or decreases the applied pressure while the pressure signal is measured, and wherein the measured pressure signal is indicative of blood pulsations and comprises a plurality of pressure pulses,
- a processor configured to determine a blood pressure determination curve based on the plurality of pressure pulses, wherein the blood pressure determination curve is indicative for a dependence of the pressure pulses on the applied pressure, and wherein the processor is further configured to apply a transformation to the blood pressure determination curve and to determine the blood pressure based on a result of the transformation.

It has been found that the manner in which the measured pressure signal is interpreted is critical for determining an accurate blood pressure value for the subject. Based on this finding, it has been realized that the blood pressure can be determined more accurately if it is determined not just based on the blood pressure determination curve itself, even though it is already indicative for a dependence of the pressure pulses on the pressure applied by the pressure applicator, but by applying a transformation to the blood pressure determination curve and determining the blood pressure based on a result of the transformation.
The pressure sensor, which is configured to measure the pressure on the skin of the encased part of the subject, can be arranged inside the shell. However, the pressure sensor can also be arranged in another way for measuring the pressure on the skin of the encased part of the subject. For instance, a fluid filled pressure sensor pad can be arranged inside the shell and connected via a fluid path, i.e. via a fluid filled tubing, to a pressure sensor outside of the shell, in order to measure the pressure on the skin of the encased part of the subject.
The pressure signal providing unit can be a receiving unit configured to receive the pressure signal from, for instance, the measurement device and to provide the received pressure signal. However, the pressure signal providing unit can also be a storage in which a previously measured pressure signal has been stored and from which it can be retrieved for providing the same. The pressure signal providing unit also can be or comprise the measurement device which measures the pressure signal.
The pressure applied by the pressure applicator to the shell and thereby to the encased part of the subject while the pressure signal is measured can be, for instance, continuously increased or decreased during a measurement time period, particularly with a constant gradient. The measured pressure signal can typically be understood as a composition, particularly a sum, of a part that is due to the increasing or decreasing applied pressure and a part that is due to blood pulsations in the part of the subject encased by the shell. Hence, the measured pressure signal comprises pressure pulses, wherein specifically the pressure pulses can be understood as being indicative of blood pulsations.
The part of the measured pressure signal that is due to the blood pulsations will typically first increase when the applied pressure is increased from near diastolic blood pressure or even below and start to decrease at some point when the applied pressure is increased further towards systolic pressure or even beyond, since, on the one hand, the applied pressure provides the necessary coupling between the blood pulsations in the part of the subject encased by the shell and the pressure sensor, but, on the other hand, tends to suppress the blood pulsations. Likewise, the part of the measured pressure signal that is due to the blood pulsations will typically also increase when the applied pressure is decreased from near systolic pressure or even above, and start to decrease at some point when the applied pressure is decreased further towards diastolic pressure or even below. This dependence, which is a dependence of the pressure pulses in the measured pressure signal on the applied pressure, is indicated also by the blood pressure determination curve determined by the processor. For instance, also the blood pressure determination curve can increase and thereafter start to decrease again in the measurement time period, particularly irrespective of whether the applied pressure is increased or decreased. The dependence of the pressure pulses in the measured pressure signal on the applied pressure, i.e. how the pressure pulses depend on the applied pressure, is indicative for blood pressure. Since this dependence is indicated by the blood pressure determination curve, also the blood pressure determination curve will be indicative of blood pressure.

The blood pressure determination curve, which the processor is configured to determine based on the plurality of pressure pulses in the pressure signal, is preferably a continuous curve, particularly an at least once differentiable curve, more particularly a smooth curve, wherein it is understood that the terms "continuous", "differentiable" and "smooth" can have an approximate, or "discretized", meaning as known from numerical analysis. The blood pressure determination curve preferably corresponds to a function of the applied pressure, the mean measured pressure or the measurement time. In fact, the processor is not necessarily configured to determine an actual graphical representation corresponding to the blood pressure determination curve. Instead, the blood pressure determination curve may just correspond to, particularly consist of, pressure values, which could be understood as blood pressure determination curve values, associated with respective values of the applied pressure, the mean measured pressure or the measurement time, for instance.

The transformation applied to the blood pressure determination curve may be predefined such that regions of the blood pressure determination curve indicating a dependence of the blood pressure pulses on the applied pressure beyond a predetermined strength can be detected more efficiently than in the blood pressure determination curve itself. It has been found that regions where the dependence of the blood pressure pulses on the applied pressure is strong can provide valuable information on the blood pressure. A more efficient detection of such regions may therefore be facilitated by emphasizing these regions. Since the pressure applied by the pressure applicator is increased or decreased over time, the dependence of the blood pressure determination curve on the applied pressure corresponds to a rate of change of the blood pressure determination curve. Hence, the dependence of the blood pressure determination curve on the applied pressure at a given point of the curve, i.e. its local strength, can be measured, for instance, in terms its slope at that point. It is recognized that also regions of the blood pressure determination curve with a weak, particularly vanishing, dependence on the applied pressure can provide valuable information on the blood pressure, but it has been found that they are not the only ones. The relative terms "strong" and "weak", referring to the strength of the dependence of the blood pressure determination curve on the applied pressure, are understood as being defined with respect to one or more predetermined thresholds. Moreover, the dependence of the blood pressure determination curve on the applied pressure, particularly the slope of the curve, may be considered in terms of its actual value, i.e. including signs, or its absolute value.

Applying the transformation to the blood pressure determination curve refers to transforming at least a part of, particularly the whole, blood pressure determination curve. Hence, the result of applying the transformation to the blood pressure determination curve may be a transformed blood pressure determination curve, wherein, if the transformation is only applied to a part of the blood pressure determination curve, the transformed blood pressure determination curve may deviate from the blood pressure determination curve only in this part, or may correspond only to this deviating part. If the blood pressure determination curve is transformed only partially, the processor may be configured to determine the blood pressure only based on the transformed part of the blood pressure determination curve. The blood pressure determination curve may be transformed only partially in case it can be anticipated which part of the blood pressure determination curve, after being transformed, is a good basis for determining the blood pressure. Restricting the applied transformation to a part of the blood pressure determination curve can save computational resources.

Although the result of applying the transformation to the blood pressure determination curve may be thought of as a transformed blood pressure determination curve, the processor is not necessarily configured to actually determine a transformed blood pressure determination curve. Instead, for instance, the processor may be configured to determine new pressure values based on the pressure values corresponding to the blood pressure determination curve, wherein these new pressure values may be referred to as transformed blood pressure determination curve values, and to determine the blood pressure based on the new pressure values. Since applying the transformation to the blood pressure determination curve may correspond to determining new pressure values based on pressure values corresponding to the blood pressure determination curve, applying the transformation may also be understood as applying a function to the blood pressure determination curve, i.e. the blood pressure determination curve values. However, it is to be noted that this function is not necessarily an ordinary function, such as an analytical function. Instead, the applied transformation may only be expressible by an operator, such as a differential operator, for instance. In a preferred embodiment the transformation applied to the blood pressure determination curve refers to taking a derivative of the blood pressure determination curve, such that the processor is configured to determine the blood pressure based on the derivative of the blood pressure determination curve. The derivative of the blood pressure determination curve has been found to be particularly helpful in indicating regions of strong dependence of the blood pressure pulses on the applied pressure and hence in determining the blood pressure more accurately, while at the same time requiring acceptable computational efforts to be computed. It is understood that, when the transformation is a derivative, the blood pressure determination curve is determined such that it is differentiable.

Instead of determining the derivative, which allows for an efficient detection of regions in the blood pressure determination curve indicating a strong dependence of the pressure pulses on the applied pressure, also differences between points of the blood pressure determination curve may be determined. The term "differences" can particularly be understood as referring to differences that are finite beyond the degree considered in numerical analysis for approximating derivatives. Alternatively, however, taking differences between different points of the blood pressure determination curve may also be understood as corresponding to determining a finite, i.e. discretized, version of a derivative, as known from numerical analysis. In any case, also a determination of differences may be understood as a transformation being applied to the blood pressure determination curve, since the determined differences may be understood as being associated with points of the blood pressure determination curve, thereby giving rise to a map mapping the blood pressure determination curve, or a part thereof, to a new curve, which could be regarded as a transformed blood pressure determination curve.

Preferentially, the processor is configured to determine a position of a maximum of the derivative of the blood pressure determination curve and to determine the blood pressure based on the determined position and the measured pressure. In particular, the processor can be configured to determine the blood pressure based on the mean of the measured pressure at the determined position of the maximum. It has been found that determining the blood pressure based on the determined maximum of the derivative of the blood pressure determination curve and depending on the measured pressure on the skin, i.e. depending on the tissue pressure, allows for a particularly accurate determination of the blood pressure. When determining the blood pressure based on the maximum of the derivative of the blood pressure determination curve, the processor may be configured to determine the derivative of the blood pressure determination curve only for a region in which the maximum of the derivative is anticipated to lie. This anticipated region may be a fixed region that is predetermined based on previous measurements.

The processor may further be configured to determine a position of a maximum of the blood pressure determination curve and to determine the blood pressure based further on this further determined position and the measured pressure. In particular, the processor can be configured to determine the blood pressure based further on the mean of the measured pressure at the determined further position, i.e. the determined position of the maximum of the blood pressure determination curve. Determining the blood pressure based on the determined maximum of the blood pressure determination curve in addition to the determined maximum of its derivative, and depending on the measured pressure on the skin, i.e. depending on the tissue pressure, allows for determining the blood pressure yet more accurately, even though the dependence of the blood pressure determination curve on the applied pressure at its maximum would ordinarily be considered as relatively weak or even vanishing, since the derivative of the blood pressure determination curve vanishes at its maximum. In fact, the processor may be configured to determine the position of the maximum of the blood pressure determination curve based on its derivative, i.e. based on the transformed blood pressure determination curve, as well, namely by determining where the derivative is zero. Alternatively, for instance, the processor may be configured to determine the position of the maximum of the blood pressure determination curve and/or its transformed version by comparing points on the respective curve, or corresponding functional values, with each other, wherein it may be determined that the respective maximum is located where the respective curve, or its corresponding function, changes from ascending, or increasing, to descending, or decreasing. Moreover, the respective maximum may be determined while the pressure signal is measured, particularly while the part of the pressure signal is measured that gives rise to the respective maximum in the respective curve.

While determining the blood pressure based on the blood pressure determination curve in addition to the transformed version of the blood pressure determination curve, particularly based on the positions of the maxima of both of these curves, may allow for a more accurate determination of blood pressure, determining the blood pressure based only on the transformed version of the blood pressure determination curve, particularly based on the position of the maximum of the derivative of the blood pressure determination curve only, can be computationally more efficient. Moreover, since the derivative of the blood pressure determination curve will usually assume its maximum earlier in the measurement time period than the blood pressure determination curve itself, this also allows for a faster blood pressure determination and therefore less discomfort for the subject.

Additionally or alternatively to determining the blood pressure based on the position of the maximum of the derivative of the blood pressure determination curve, the blood pressure may be determined based on the position of the minimum of the derivative of the blood pressure determination curve. This may particularly be advantageous in case the measurement time during which the pressure applicator increases or decreases the applied pressure and the pressure signal is measured is relatively long.

As already indicated above, the blood pressure determination curve can particularly correspond to a function of the applied pressure or the mean measured pressure. Accordingly, instead of the measured pressure, particularly its mean, also an applied pressure at the respective maxima could be used for determining the blood pressure. The applied pressure may refer to a pressure which the pressure applicator is controlled to apply.

It is understood that it might still be acceptable to not use the respective pressure value exactly at the determined position of the respective maximum for determining the blood pressure, but instead a pressure close to this pressure. In other words, it might be acceptable if the respective maximum is not precisely determined, but with a predetermined sufficient accuracy.

In an embodiment the processor is configured to determine the blood pressure as a function of a) the mean of the measured pressure at the determined position of the maximum of the derivative of the blood pressure determination curve and b) the mean of the measured pressure at the determined position of the maximum of the blood pressure determination curve, wherein the dependence of the function on the mean of the measured pressure at the two positions is predetermined based on reference measurements. In particular, the function can be a linear function whose dependence on the mean of the measured pressure at the two positions is expressed by linear coefficients, wherein the linear coefficients are predetermined in a linear regression carried out based on the reference measurements. While generic functions may allow for yet more accuracy, a linear function keeps the computational effort acceptable while still allowing for a very high accuracy in determining the blood pressure.

The reference measurements, which could also be referred to as calibration measurements, preferably correspond to an evaluation of a calibration set consisting of pairs of a an) invasively measured blood pressure values and b) noninvasive blood pressure values determined based on noninvasively measured pressure signals, wherein the noninvasively measured pressure signals are measured by an apparatus as defined above simultaneously to the invasive measurement. The calibration set is preferably collected from an adequate number of individuals and in different hemodynamic conditions. The noninvasive blood pressure values are determined by the apparatus as defined above, and the linear coefficients of the above indicated function expressing the blood pressure in terms of the applied pressure, particularly the mean of the measured pressure at the two positions indicated above, can be optimized such that deviations between invasive blood pressure values and noninvasive blood pressure values are minimized. For instance, a linear regression using the least squares method can be carried out.

While it has been found to allow for a very accurate determination of blood pressure to determine the blood pressure based on the derivative of the blood pressure determination curve, also other transformations being applied to the blood pressure determination curve may lead to a good, or even better, basis for an accurate determination of blood pressure. For instance, the processor may be configured to determine a logarithm of the blood pressure determination curve and determine the blood pressure based thereon.

Moreover, while the term "derivative" has been used above and will also subsequently be used to refer to the first derivative, also higher derivatives could be used as transformations to be applied to the blood pressure determination curve.

Preferentially, the processor is configured to determine, for a respective pressure pulse of the plurality of pressure pulses, at least one feature that characterizes the respective pressure pulse, to determine, for the respective pressure pulse, a blood pressure determination value based on the determined at least one feature such that for several pressure pulses, which are present at different times, several blood pressure determination values are determined, wherein the processor is configured to determine the blood pressure determination curve such that the several blood pressure determination values determined for the several pressure pulses and hence for several times form the blood pressure determination curve. The blood pressure determination curve used in this case for the blood pressure measurement could be regarded as being constructed from individual tissue pressure waveforms (TPW), such that it can be abbreviated as TPW_M-curve, while the blood pressure determination values could be regarded as parametrizing the tissue pressure waveforms, such that they can be abbreviated as TPWP_M. It has been found that, even if a single feature is considered for determining the blood pressure determination values, it is possible to accurately determine the blood pressure. It hence is possible to accurately determine the blood pressure with relatively low computational efforts.

The at least one feature can directly characterize the respective pressure pulse or indirectly characterize the respective pressure pulse. In the latter case the respective pressure pulse is processed for determining a feature determination pulse and the at least one feature is determined based on the determined feature determination pulse. This will be explained in more detail further below. The processor can be configured to determine, for each pressure pulse of all or of only some, for instance, at least 5 or at least 10, of the plurality of pressure pulses, the at least one feature that characterizes the respective pressure pulse.

The processor can be adapted to process the several blood pressure determination values, which are obtained for the several pressure pulses, for obtaining the blood pressure determination curve. For instance, an interpolation can be applied and optionally also a smoothing. In this way, a continuous, at least once differentiable or even smooth blood pressure determination curve can be obtained.

In an embodiment, the processor is configured to, in order to determine for a respective pressure pulse the at least one feature, provide a feature determination pulse based on the respective pressure pulse, and determine at least one of the following features: i) the difference (TPP) between the feature determination pulse's maximum systolic pressure (TPsys) and the feature determination pulse's pressure at the end-diastolic point (TPdia), ii) the area (TPA+.top50) enclosed by an upper part of the feature determination pulse, wherein a) the upper end of the upper part is at the feature determination pulse's maximum systolic pressure (TPsys) and b) the lower end of the upper part is between the feature determination pulse's maximum systolic pressure (TPsys) and a pressure value which corresponds to a mean (TPcl) of the measured pressure (TP), iii) the duration (t(pulse)) of the respective feature determination pulse, iv) the area (TPA/TPA.norm) of the respective feature determination pulse, and v) the width at half maximum (W50) of the respective feature determination pulse. It has been found that by using at least one of these features the determination of the blood pressure can be further improved.

The feature determination pulse for a respective pressure pulse can be obtained, for instance, by subtracting a mean measured pressure (TPcl) from the respective pressure pulse. However, the feature determination pulse for a respective pressure pulse can also be determined in another way. It can also directly be the respective pressure pulse, i.e. the respective measured pressure pulse. If the feature determination pulse for a respective pressure pulse has been obtained by subtracting a mean measured pressure (TPcl) from the respective pressure pulse, for determining the area TPA+.top 50 the pressure value, which corresponds to the mean (TPcl) of the measured pressure (TP), would be zero.

The difference (TPP) between the feature determination pulse's maximum systolic pressure and the feature determination pulse's pressure at the end-diastolic point preferentially is the difference between the maximum measured pressure and the minimum measured pressure of the respective pressure pulse, i.e. the difference between the tissue pressure (TP), i.e. the measured pressure on the skin, of the respective pressure pulse at a maximum systolic point and the tissue pressure (TP) at an end diastolic point of the respective pressure pulse. The duration t(pulse) of the respective feature determination pulse preferentially corresponds to the time difference between an end-diastolic point and the following end-diastolic point for the respective pressure pulse. Moreover, the area TPA is preferentially the area under the curve of the respective feature determination pulse from an end diastolic point to the following end diastolic point. The area can be a normalized area and is then named "TPA.norm". For instance, the area TPA can be normalized by scaling it to the difference between the maximum pressure and the minimum pressure of the respective feature determination pulse, in order to determine TPA.norm. The width W50 at half maximum corresponds to the width at 50 percent of the difference between the maximum pressure and the minimum pressure of the respective feature determination pulse. Thus, it is the width at 50 percent of the difference between the pressure of the feature determination pulse at the maximum systolic point and the pressure of the feature determination pulse at an end diastolic point.

In a preferred embodiment the processor is configured to determine the area TPA+.top50 enclosed by the upper part of the feature determination pulse such that the lower end of the upper part is at a pressure value which corresponds to half of the pressure distance (TPP+) between the feature determination pulse's maximum systolic pressure (TPsys) and the pressure value which corresponds to the mean (TPcl) of the measured pressure (TP).

Moreover, in a preferred embodiment the processor is configured to determine the blood pressure determination values (TPWP_M) and hence form the blood pressure determination curve (TPW_M-curve) only based on the feature being the difference (TPP) between the feature determination pulse's maximum systolic pressure (TPsys) and the feature determination pulse's pressure at the end-diastolic point (TPdia). Thus, in this embodiment, besides TPP, no other of the above mentioned features is used for determining the blood pressure determination values. It has been found that this allows for a further increased accuracy of determining the blood pressure with relatively low computational efforts.

In a further improved embodiment the processor is configured to determine the blood pressure determination values (TPWP_M) and hence form the blood pressure determination curve (TPW_M-curve) only based on a) the feature being the difference TPP between the feature determination pulse's maximum systolic pressure (TPsys) and the feature determination pulse's pressure at the end-diastolic point (TPdia) and b) the feature being the area TPA+.top50 enclosed by the upper part of the feature determination pulse. Thus, in this embodiment, besides TPP and TPA+.top50, no other of the above mentioned features is used for determining the blood pressure determination values. It has been found that this allows for an even further increased accuracy of determining the blood pressure, wherein still the computational efforts can be relatively low.

Preferentially the processor is configured to provide a function which receives as input the at least one feature of a respective pressure pulse and which outputs a respective blood pressure determination value, and to determine the blood pressure determination curve such that it is formed by the respective blood pressure determination value together with the blood pressure determination values determined for the other pressure pulses. The function has at least one parameter which can be determined by calibration, wherein by invasive means reference blood pressure values are determined very accurately and the at least one parameter is determined such that the apparatus yields the very accurately invasively measured blood pressure values with high statistical precision and accuracy. In particular, for the calibration measurement pairs of simultaneously recorded invasive and noninvasive blood pressure values from an adequate number of individuals in different hemodynamic conditions are used. It should be noted that the calibration is preferentially only carried out in a development phase, i.e. not during an actual blood measurement procedure. The calibration can be carried out separately for different shell sizes, i.e. preferentially for different blood pressure cuff sizes, or for different groups of shell sizes. For instance, for different shell sizes or groups of shell sizes, different parameters of the function can be determined by calibration, i.e. for each shell size or for each group of shell sizes a respective at least one parameter can be determined.

The processor may also be configured to control the measurement of the pressure signal, possibly depending on determined blood pressure values, i.e. blood pressure values determined during the measurement. In an embodiment the processor is configured to control the measurement device such that the applied pressure is increased in a measurement time period extending till an end measurement time point and the applied pressure is decreased in a following post-blood-pressure-measurement time period, wherein the pressure sensor measures the pressure (TP) on the skin at least during the measurement time period, wherein the processor is configured to determine an end measurement time point, at or after which the measurement time period is to be stopped, based on the at least one feature determined for at least some of the plurality of pressure pulses, and to control the pressure applicator such that, when or after the end measurement time point has been reached, it decreases the applied pressure to start the following post-blood-pressure-measurement time period. In particular, the at least one feature respectively determined for the pressure pulses can be used for determining an end measurement time point such that the measurement time period is relatively short and nevertheless enough pressure data have been measured, which allow for an accurate determination of the blood pressure. This allows for a reduced blood pressure measurement time and tissue pressure, i.e. the clamping pressure exerted on the skin of the body part, during measurement staying and ending substantially below systolic arterial pressure.

Preferentially, the processor is configured to determine a) for a respective pressure pulse an end determination value based on the at least one feature that has been determined for the respective pressure pulse, such that for several pressure pulses, which are present at different times, several end determination values are determined, wherein the several end determination values determined for the several pressure pulses and hence for the several times form an end determination curve, and b) the end measurement time point based on the end determination curve. By using the end determination curve the accuracy of determining the end measurement time point can be further increased. The processor can be adapted to process the several end determination values, which are obtained for the several pressure pulses, for obtaining a continuous end determination curve. For obtaining the continuous curve based on the distinct end determination values known mathematical techniques can be used. For instance, an interpolation can be applied, a fitting procedure, a filtering procedure, et cetera.

The processor is preferentially configured to determine the end determination curve such that it fulfills one of the following conditions: a) a maximum of the end determination curve occurs temporally before a maximum of the blood pressure determination curve, b) a maximum of the end determination curve occurs temporally at or after a maximum of the blood pressure determination curve and the decrease of the end determination curve after its maximum is steeper than the decrease of the blood pressure determination curve after its maximum, and c) the end determination curve is identical to the blood pressure determination curve. It has been found that, if the end determination curve and the blood pressure determination curve have one of these relations with respect to each other, the end measurement time point can be even more accurately determined.

In a preferred embodiment, the processor is configured to determine the end measurement time point further based on the blood pressure determination curve. It has also been found that, if not only the end determination curve, but also the blood pressure determination curve, are used for determining the end measurement time point, the measurement time can be even further reduced.

Preferentially the processor is configured to determine the end measurement time point by determining when a) the end determination curve has fallen, after having passed its maximum, to a value being equal to or smaller than a predefined percentage of the maximum and b) the blood pressure determination curve has reached or passed its maximum. It is further preferred that the predefined percentage of the maximum is within a range from 40 percent to 95 percent. It has especially been found that, if both curves are used in this way for determining the end measurement time point, this time point can be determined very accurately such that the blood pressure measurement time is relatively short and blood pressure is nevertheless measured very precisely.

In an embodiment, the processor is configured to control the pressure applicator such that it increases the applied pressure with a first rate in a pre-measurement time period which is followed by the measurement time period in which the applied pressure is increased with a second rate, wherein the first rate is larger than the second rate. Thus, an applied pressure, at which the measurement time period should start, can be reached relatively fast, thereby allowing for a further reduction of the overall time needed for measuring the blood pressure.

In an embodiment the processor is configured to control the pressure applicator such that at the end of the pre-measurement time period the measured pressure is within the range of 15 to 30 mmHg. This ensures that during the measurement time period the pressure on the skin, i.e. the tissue pressure, is measured over a sufficiently large pressure range, which allows for an accurate and precise measurement of the blood pressure.

The range of 15 to 30 mmHg is preferentially applied, if a previous diastolic arterial pressure obtained by a previous blood pressure measurement of the same subject is not used by the processor for controlling the pressure applicator. If a previous diastolic arterial pressure is used for the control of the pressure applicator, the measured pressure at the end of the pre-measurement time period can also be higher. The control of the pressure applicator depending on a previously obtained diastolic arterial pressure will be explained below.

It should be noted that the pressure at the end of the pre-measurement time is not the pressure which is applied in between two subsequent blood pressure measurements, particularly before the pre-measurement time period starts. This pressure could be named "attachment pressure" and should not exceed 15 mmHg.

In an embodiment the processor is configured to a) store or receive a previous diastolic arterial pressure obtained by a previous blood pressure measurement, b) determine a first end measured pressure, which should be present at the end of the pre-measurement time period and which could therefore also be regarded as being a targeted pressure, depending on the previous diastolic arterial pressure, such that the first end measured pressure is smaller than the previous diastolic arterial pressure, and c) control the pressure applicator such that the measured pressure at the end of the pre-measurement time period is equal to or smaller than the determined first end measured pressure. It is preferred that the processor is configured to determine the first end measured pressure such that it is 90 percent or less of the previous diastolic arterial pressure. In addition, this ensures that during the measurement time period the pressure on the skin, i.e. the tissue pressure, is measured over a sufficiently large range to determine blood pressure accurately and precisely.

In an embodiment the processor is configured to further store or receive the time at which the previous diastolic arterial pressure had been measured and to determine the first end measured pressure depending on i) the previous diastolic arterial pressure and ii) a temporal distance to the blood pressure measurement, at which the previous diastolic arterial pressure had been measured, as indicated by the stored time. The diastolic arterial pressure can change with time, wherein the first end measured pressure can still be determined relatively accurately, if the temporal distance to the blood pressure measurement, at which the previous diastolic arterial pressure has been measured, is considered. Thus, although this uncertainty regarding the change of the diastolic arterial pressure over time is present, the end of the pre-measurement time period can be determined such that, during the following measurement time period, pressure on the skin, i.e. tissue pressure, is measured over a sufficiently large range to determine blood pressure accurately and precisely.

The processor can be further configured to a) store or receive a previous pressure measured by the pressure sensor in a time period in between an end of a previous measurement time period of a previous blood pressure measurement and a start of the pre-measurement time, and b) to control the pressure applicator such that the measured pressure at the start of the pre-measurement time period is equal to or smaller than a predefined pressure value based on the stored or received previous measure. The predefined pressure value is preferentially equal to or smaller than 15 mmHg, further preferred equal to or smaller than 10 mmHg.

The measured pressure at the start of the pre-measurement time period can be regarded as being the attachment pressure which is present during measurement pauses between two subsequent blood pressure measurements, i.e. before a respective blood pressure measurement starts, wherein this attachment pressure is preferentially controlled to achieve a given or pre-set value, i.e. the predefined pressure value. This control is preferentially carried out to adapt for changes in body part volume, e.g. increases caused by tissue edema (capillary leak like e.g. in sepsis, hypervolemia) or decreases by shrinking tissue edema or by hypovolemia. This should especially avoid venous congestion during measurement pauses, i.e. in between two subsequent blood pressure measurements, caused by compromised venous return by venous compression with tissue pressures higher than venous pressure in the respective body part. In a preferred embodiment the pressure on the skin, which can also be regarded as being tissue pressure, is continuously measured during the entire procedure of carrying out one or several blood pressure measurements and the pressure applicator is controlled such that venous congestion is avoided during measurement pauses.

In another aspect of the present invention a method for determining blood pressure of a subject is presented, wherein the method includes:

- providing a measured pressure signal (TP) of a subject over a time by a pressure signal providing unit, wherein the pressure signal (TP) has been measured by using a measurement device comprising a) a shell configured to encase a part of the subject, through which blood flows, b) a pressure applicator configured to apply, from outside the shell, pressure to the shell and thereby to the encased part of the subject, and c) a pressure sensor configured to measure the pressure signal on the skin of the encased part of the subject, wherein the pressure applicator increases or decreases the applied pressure while the pressure signal is measured, and wherein the measured pressure signal is indicative of blood pulsations and comprises a plurality of pressure pulses,
- determining a blood pressure determination curve based on the plurality of pressure pulses, wherein the blood pressure determination curve is indicative for a dependence of the pressure pulses on the applied pressure,

- applying a transformation to the blood pressure determination curve, and
- determining the blood pressure based on a result of the transformation.

In a further aspect of the present invention a computer program for determining blood pressure is presented, wherein the computer program comprises program code means for causing the apparatus for determining blood pressure to carry out the steps of the method for determining blood pressure.

The apparatus, method and computer program can be adapted to determine the blood pressure continuously, i.e. several subsequent blood pressure measurements are carried out, or to determine the blood pressure non-continuously, i.e. for instance one time.

It shall be understood that the processor of claim 1, the method of claim 14, and the computer program of claim 15, have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

BRIEF DESCRIPTION OF THE DRAWINGS

In the following drawings:

Fig. 1 shows schematically and exemplary an embodiment of an apparatus for determining blood pressure of a subject, which comprises a measurement device, in a situation in which a blood pressure cuff of the measurement device is inflated,

Fig. 2 shows schematically and exemplary a shell of the measurement device encasing an upper arm of the subject,

Fig. 3 shows schematically and exemplary the apparatus in a situation in which the blood pressure cuff is deflated,

Fig. 4 shows schematically and exemplary a measured tissue pressure and further values derived from the tissue pressure measurement,

Figs. 5 to 8 illustrate calculations of different features for a pressure pulse of the measured tissue pressure,

Fig. 9 shows schematically and exemplary an end determination curve and a blood pressure determination curve,

Fig. 10 illustrates schematically and exemplary a determination of blood pressure values by using the blood pressure determination curve,

Fig. 11 shows schematically and exemplarily several curves used for blood pressure determination, including a curve corresponding to a derivative of the blood pressure determination curve,

Fig. 12 shows schematically and exemplary an example of several successive blood pressure measurements,

Fig. 13 shows schematically and exemplary a further example of several successive blood pressure measurements, and

Fig. 14 shows a flowchart exemplary illustrating an embodiment of a method for determining blood pressure of a subject.

DETAILED DESCRIPTION OF EMBODIMENTS

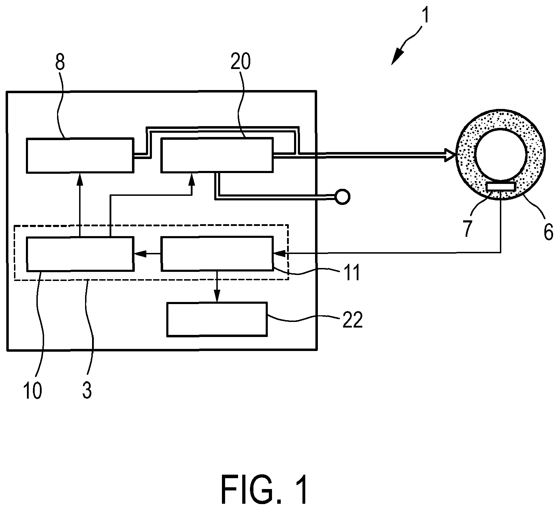

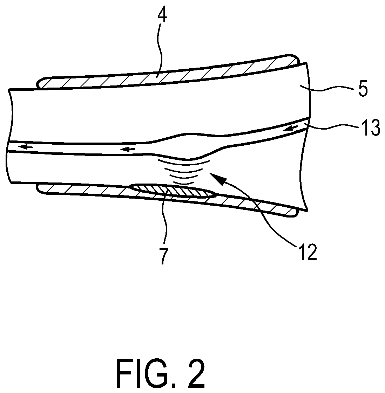

Fig. 1 shows schematically and exemplarily an apparatus 1 for determining blood pressure of a subject. The apparatus 1 comprises a shell 4 which can be seen in Fig. 2 and which is configured to encase a part 5 of the subject, through which blood flows. In this embodiment the part 5 of the subject is an arm of the subject, wherein in Fig. 2 within the arm 5 a brachial artery 13 is shown and wherein the arrows within the brachial artery 13 indicate the flow direction of the blood away from the heart. The apparatus 1 further comprises a pressure sensor 7 arranged inside the shell 4 and configured to measure the pressure on the outer skin of the encased arm 5 of the subject. The measured pressure can also be regarded as being tissue pressure (TP). As indicated in Fig. 2, a pulse wave within the brachial artery 13 causes a pressure wave 12 which is transmitted to the pressure sensor 7 via the tissue of the arm 5. The measured pressure signal will therefore comprise pressure pulses indicative of blood pulsations. The shell 4 is not shown in Fig. 1 for clarity reasons.

The apparatus 1 further comprises a cuff 6 which encloses the shell 4 and which is inflatable by using a pump 8 for applying, from outside the shell 4, pressure to the shell 4 and thereby to the encased arm 5 of the subject. Since the cuff 6 and pump 8 together enable an application of the pressure to the shell 4 and thereby to the encased arm 5 of the subject, they can be regarded as forming a pressure applicator 6, 8. Moreover, the shell 4, the pressure applicator 6, 8 and the pressure sensor 7 can be regarded as being components of a measurement device which is controlled by a processor 3. The shell 4 with the cuff 6 is preferentially a kinking-proof shell cuff as described in WO 2014/121945 A1.

The processor 3 is configured to control the measurement device such that the pressure applicator 6, 8 increases the applied pressure in a measurement time period extending till an end measurement time point and decreases the applied pressure in a following post-blood-pressure-measurement time period, and such that the pressure sensor 7 measures the pressure on the skin, i.e. the tissue pressure TP, at least during the measurement time period. Moreover, the processor 3 is configured to control the pressure applicator 6, 8 such that it increases the applied pressure with a first rate in a pre-measurement time period which is followed by the measurement time period in which the applied pressure is increased with a second rate, wherein the first rate is larger than the second rate. The control of the measurement device such that the cuff 6 is inflated and hence the applied pressure is increased is illustrated in Fig. 1, i.e. the bold arrows indicate the inflation situation.

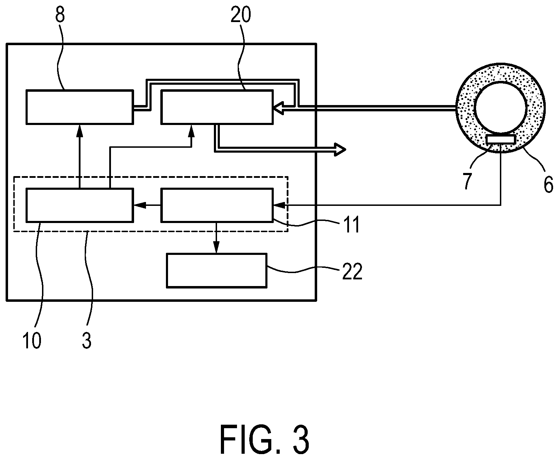

The apparatus 1 comprises a valve 20 which, when the valve 20 is opened, allows the compressed air within the system to leave the system into the surrounding atmosphere for deflating the cuff. In Fig. 3 this deflation situation, in which the pump 8 is switched off, is indicated by the bold arrows.

The processor 3 can be regarded as comprising a controlling part 10 for controlling the pump 8 and the valve 20 and a processing part 11, which especially is configured to carry out some calculations that will be explained further below. The apparatus 1 can also comprise a display 22 for displaying, for instance, a measured blood pressure value. While in this embodiment the processor 3 also controls the measurement, wherein the controlling part 10 may determine how to control the measurement based on an output of the processing part 11, in other embodiments the processor may not be used to control the measurement, but instead, for instance, to process the measurement data after the measurement has been completed.

In the pre-measurement time period, which could also be regarded as being a fast inflation period, the processor 3 controls the apparatus 1 such that the valve 20 is closed and the pump 8 inflates the cuff 6 with the larger first rate. In the following measurement time period the processor 3 also controls the apparatus 1 such that the valve 20 is closed, but the pump 8 is controlled such that the inflation of the cuff 6 is continued with the lower second rate. The measurement time period could therefore also be regarded as being a slow inflation period.

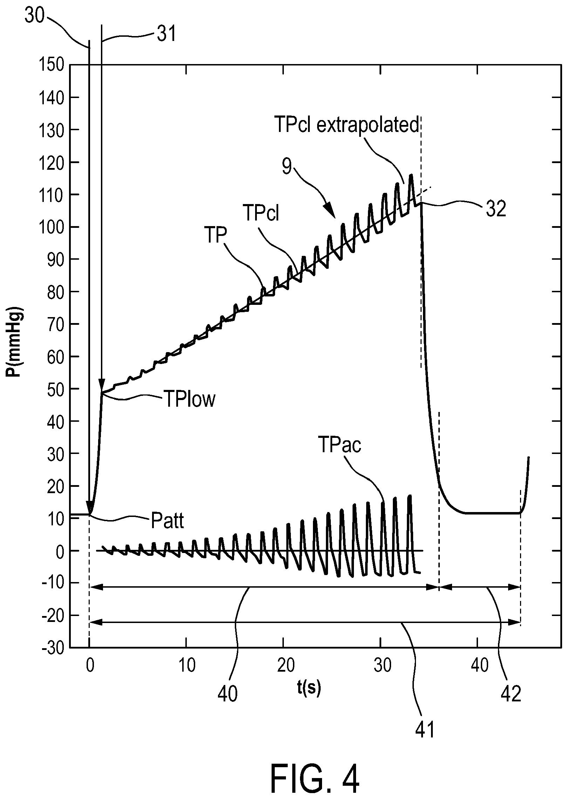

Fig. 4 schematically and exemplarily illustrates the measured pressure TP versus time t. The first inflation in the pre-measurement time period starts with a tissue pressure TP being an attachment pressure Patt that is the measured tissue pressure in the uninflated cuff 6. This attachment pressure Patt can range, for instance, from 0 to 15 mmHg. An attachment pressure Patt up to 15 mmHg has turned out to not result in venous congestion for more than 12 hours, thereby making the assembly of the shell 4 with the cuff 6 optimally suitable for longer term monitoring. For this reason, it is preferred that the attachment pressure Patt is not larger than 15 mmHg. In Fig. 4 the arrow 30 indicates the start of the pre-measurement time period, i.e. the start of the fast inflation period. During this pre-measurement time period the part of the overall tissue pressure range, which has no or substantially no information for the determination of the blood pressure, shall be passed as fast as possible. Therefore, the inflation rate is preferentially as large as possible during the pre-measurement time period. For instance, the inflation rate with respect to the tissue pressure TP can be equal to or larger than 8 mmHg/s. This pre-measurement time period with a fast inflation rate ends at a tissue pressure value which is indicated in Fig. 4 by "TPlow".

The tissue pressure value TPlow also indicates the start of the measurement time period with the slower second inflation rate. In Fig. 4 this start of the measurement time period, which can also be regarded as being a slow inflation period, is indicated by the arrow 31. In an embodiment, the processor 3 is configured to control the pump 8 such that at the end of the pre-measurement time period the measured pressure TPlow is within a range of 15 to 30 mmHg. Thus, in an embodiment the tissue pressure TPlow can be predefined such that it is a value between 15 and 30 mmHg. However, in a situation, in which a diastolic arterial pressure determined in a preceding measurement (DAPprev) is available, the tissue pressure TPlow can depend on this diastolic arterial pressure and the tissue pressure TPlow can also be larger. This larger TPlow is schematically shown in Fig. 4. For instance, the processor 3 can be configured to store (or access a memory storage with) a previous diastolic arterial pressure DAPprev. The previous diastolic arterial pressure DAPprev has been obtained by a previous blood pressure measurement and can be used to determine the tissue pressure TPlow. The tissue pressure TPlow should be present at the end of the pre-measurement time period, i.e. of the first inflation period, and could therefore also be regarded as being a first end measured pressure. The tissue pressure TPlow is determined depending on the previous diastolic arterial pressure DAPprev such that the tissue pressure TPlow is smaller than the previous diastolic arterial pressure DAPprev. Preferentially, the processor is configured to control the pump 8 such that the measured pressure at the end of the pre-measurement time period is equal to the determined first end measured pressure TPlow. In an embodiment the processor 3 determines the tissue pressure TPlow at the end of the pre-measurement time period such that it is not above a predetermined percentage of the previous diastolic arterial pressure DAPprev. This can ensure that all necessary tissue pressure pulse curves, which allow for an accurate calculation of the blood pressure, are recorded. The calculation of the blood pressure based on the measured tissue pressure will be explained further below. The predetermined percentage is preferentially 90 percent.

The blood pressure can change between measurements due to, for instance, clinically relevant events occurring to the subject. It has been found that the diastolic arterial pressure (DAP) might decrease by about 30% within one minute in most severe situations of acute blood loss during surgery. In order to cope with these possible blood pressure changes, the processor 3 can be adapted to determine the tissue pressure TPlow depending on the previous diastolic arterial pressure DAPprev and depending on duration of a break time between measurements. Thus, the processor 3 can be configured to further store (or access the memory storage with) the time at which the previous diastolic arterial pressure DAPprev had been measured and to determine the first end measured pressure TPlow depending on i) the previous diastolic arterial pressure DAPprev and ii) a temporal distance to the blood pressure measurement, at which the previous diastolic arterial pressure DAPprev had been measured as indicated by the stored time. An exemplary specific equation for determining the first end measured pressure TPlow will be given further below.

The second inflation rate applied during the measurement time period is preferentially within a range from 1 mmHg/s to 6 mmHg/s, the lowest inflation rate preferentially at 1.5 mmHg/s, the highest preferentially 2.5 mmHg/s. It has been found that a second inflation rate within this range allows for an accurate blood pressure determination. It should be noted that these rates refer to the changes of the tissue pressure TP as measured by the pressure sensor 7.

In an embodiment the slower second inflation rate depends on the heart rate (HR) and/or the pulse pressure (PP) being the difference between the systolic and diastolic blood pressure. For instance, the second inflation rate can increase with increasing heart rate and with increasing pulse pressure PP. This increase can be linear or can be described by another functional relation. In an embodiment this functional relation is such that at a heart rate HR of 40/min and at a pulse pressure PP of 20 mmHg the tissue pressure inflation rate is 1 mmHg/s and at a heart rate of HR of 80/min and at a pulse pressure PP of 100 mmHg the tissue pressure inflation rate, i.e. the second slow inflation rate, is 10 mmHg/s. The measurement time period ends at an end measurement time point 32, thereafter an immediate fast deflation follows preferentially with a maximally possible decrease rate, because no tissue pressure data needs to be collected after the end measurement time point 32.

In Fig. 4 the time interval 40 indicates the inflation-deflation time period from the start 30 of fast inflation until the tissue pressure TP has dropped below 20 mmHg during the fast deflation, in order to allow venous return. The time period 41 indicates a cycle time being the time between a start of a measurement and a start of a following measurement and the time period 42 indicates a break period being the difference between the inflation-deflation time period 40 and the cycle time 41.

Fig. 4 also illustrates mean pressure TPcl that can be regarded as being a tissue clamping pressure affecting the tissue when the cuff 6 is attached to the arm 5, for instance, to the upper arm, of the subject. The mean pressure TPcl can be calculated by applying a low pass filter to the tissue pressure TP, wherein the low pass filter might be located within the processor 3. The low pass filter is preferentially dimensioned such that the mean pressure TPcl comprises frequencies below the lowest expected pulse rate (PRni) only. Preferred low pass filters will be described further below. The processor 3 is preferentially configured to determine an alternating component TPac of the tissue pressure by subtracting the mean pressure TPcl from the measured tissue pressure TP, i.e. TPac = TP - TPcl. In Fig. 4 the TPac curve is shown enlarged by a factor of 2, in order to improve visibility of this curve.

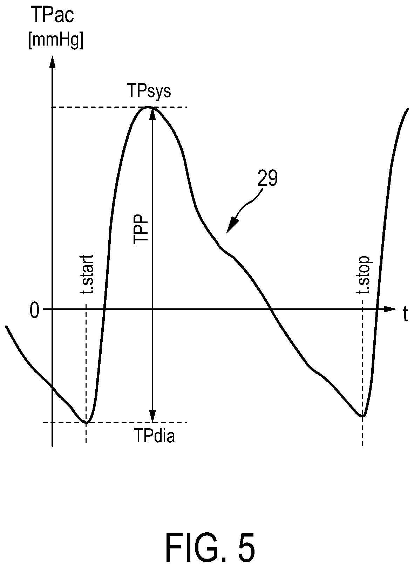

During the slow inflation period, i.e. during the measurement time period, TP pulse curves and/or TPac pulse curves are recorded, which may be analyzed simultaneously, i.e. online, wherein the TP pulse curves and/or the TPac pulse curves have tissue pressure waveforms (TPW) comprising information which allows for an accurate determination of the blood pressure. Since the blood pressure is noninvasively measured (although it nevertheless has an accuracy which is comparable to the accuracy of invasively measured blood pressure), it can be abbreviated with niBP meaning "noninvasive blood pressure". A TPac pulse curve is schematically and exemplarily shown in Figs. 5 to 8.

In the following the TPac pulse curves are used for determining features for the pressure pulses 9. The TPac pulse curves can therefore be regarded as being feature determination pulse curves or feature determination pulses. Figs. 5 to 8 hence show feature determination pulse curves or feature determination pulses 29 being TPac pulses. In another embodiment the feature determination pulses can also directly be the TP pulse curves, i.e. the pressure pulses 9 can be directly used for determining the features. The processor 3 is configured to determine for a respective pressure pulse of the plurality of pressure pulses 9 at least one feature which characterizes the respective pressure pulse. The features determined for the pressure pulses 9 are indicative for the pressure pulses 9, i.e., for instance, indicative for a shape of the pressure pulses 9 and/or pressure values characterizing the pressure pulses 9.

In an embodiment, the processor 3 is configured to determine a difference TPP between a maximum measured pressure and a minimum measured pressure of the respective feature determination pulse 29 as a feature for the respective pressure pulse. This feature will be described with reference to Fig. 5 in the following.

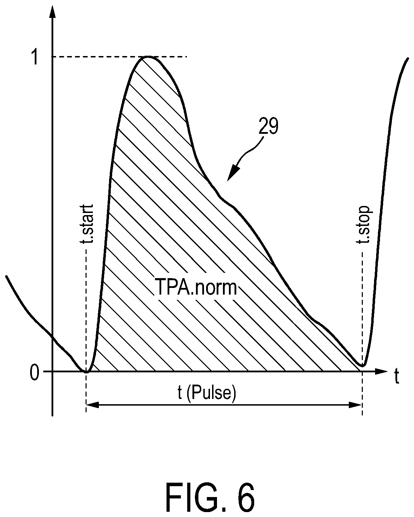

The difference TPP is a difference of a feature determination pulse's maximum systolic pressure (TPsys) and the feature determination pulse's pressure at an end-diastolic point (TPdia), which is preferably the minimum pressure of the respective feature determination pulse. In Fig. 5 and also in Figs. 6 to 8 the terms "t.start" and "t.stop" indicate the start and the end, respectively, of the respective pulse 29.

The processor 3 can also be configured to determine the pulse duration (t(Pulse)) being the time difference between an end-diastolic point and a following end-diastolic point of the feature determination pulse 29. This feature can also be defined as being the temporal difference between the start of the respective pulse (t.start) and the end of the respective pulse (t.stop). This feature is indicated in Fig. 6.

The processor 3 can also be adapted to determine the pulse area (TPA) of the respective pulse 29 being the area under the respective pulse curve within the time defined by t.start to t.stop and ranging from the feature determination pulse's pressure at an end-diastolic point (TPdia) to a feature determination pulse's maximum systolic pressure (TPsys). Preferentially, the pulse area TPA is scaled to TPP=1 as indicated in Fig. 6. This scaled pressure pulse area is named "TPA.norm".

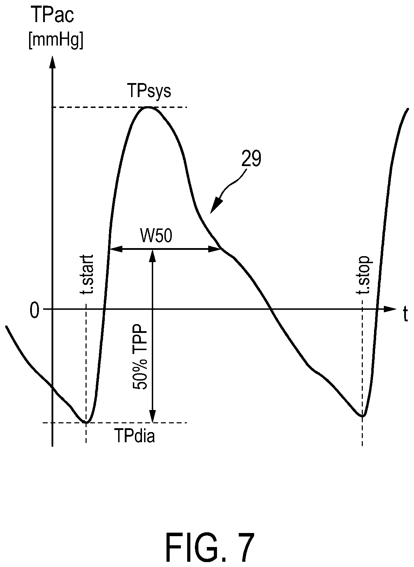

The processor 3 can also be adapted to determine the pulse width at half maximum (W50) of the respective pulse 29 as indicated in Fig. 7.

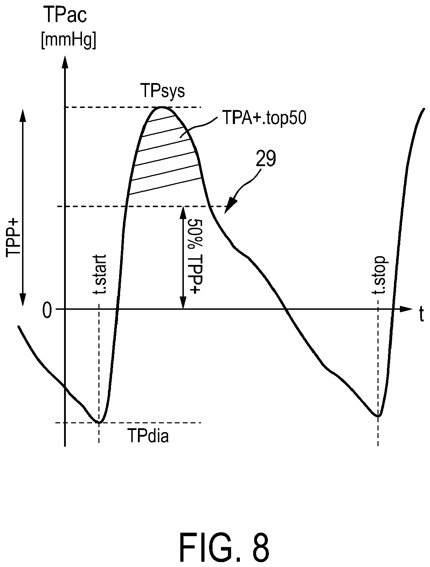

Moreover, the processor 3 can be adapted to determine the area TPA+.top50 enclosed by an upper part of the feature determination pulse 29, as schematically illustrated in Fig. 8. The upper end of the area TPA+.top50 enclosed by the upper part is at the feature determination pulse's 29 maximum systolic pressure TPsys and the lower end of the area TPA+.top50 of the upper part is between the feature determination pulse's 29 maximum systolic pressure TPsys and a pressure value which corresponds to the mean TPcl of the measured pressure TP. Since the feature determination pulse 29 has been determined by subtracting the mean TPcl from the measured pressure TP, the pressure value, which corresponds to the mean TPcl of the measured pressure TP, is zero. Preferentially, the processor 3 is configured to determine the area TPA+.top50 enclosed by the upper part of the feature determination pulse such that the lower end of the upper part is at a pressure value which corresponds to half of the pressure distance TPP+ between the feature determination pulse's maximum systolic pressure TPsys and the pressure value which corresponds to the mean TPcl of the measured pressure TP.

Figs. 5 to 8 show a TPac pulse curve such that in this example also the features have been defined based on the TPac pulse curve. However, as mentioned above, it is also possible to define these or other features based on a TP pulse curve.

The processor 3 is further configured to determine for a respective pressure pulse a blood pressure determination value TPWP M based on at least one determined feature such that for several pressure pulses, which are present at different times, several blood pressure determination values TPWP_M are determined, wherein the processor 3 is configured to determine the blood pressure determination curve TPW_M-curve such that the several blood pressure determination values TPWP_M determined for the several pressure pulses and hence for several times form the blood pressure determination curve TPW_M-curve.

The processor 3 is configured to determine the blood pressure determination values TPWP M based on the features determined for the pressure pulses 9. Hence, like the features determined for the pressure pulses 9, also the blood pressure determination values TPWP_M are indicative for the pressure pulses 9. Since the blood pressure determination curve TPW_M-curve is formed by the several blood pressure determination values TPWP M determined for the several pressure pulses 9 and hence for several times, wherein the applied pressure changes over the time, the blood pressure determination curve TPW_M-curve is indicative for a dependence of the pressure pulses 9 on the applied pressure.

The processor 3 is further configured to apply a transformation to the blood pressure determination curve TPW_M-curve and to determine the blood pressure based on a result of the transformation, i.e. based on a transformed version TPW M-curve' of the blood pressure determination curve TPW_M-curve or, if only a part of the blood pressure determination curve TPW_M-curve is transformed, based on the transformed part. In this embodiment the transformation corresponds to taking the derivative, i.e. the first derivative, such that the result of the transformation, i.e. TPW_M-curve', is the first derivative of the of the blood pressure determination curve TPW_M-curve, but in other embodiments the transformation may be a different one, wherein a possible different transformation may correspond to applying a function like a logarithm or a higher derivative, for instance, to the blood pressure determination curve TPW_M-curve. While generally this is not necessarily the case, in this embodiment the processor 3 is configured to determine the blood pressure based also on the blood pressure determination curve TPW_M-curve itself.

In particular, the processor 3 is configured to determine a position of a maximum (TPW_M-curve'.max) of the derivative TPW_M-curve' of the blood pressure determination curve TPW_M-curve and additionally a position of a maximum (TPW_M-curve.max) of the blood pressure determination curve TPW_M-curve and to determine the blood pressure based on the two determined positions and the measured pressure TP. For instance, the processor 3 is configured to determine the systolic arterial blood pressure (SAPni) based on the mean TPcl of the measured pressure TP at the two determined positions in accordance with following equation:

$$\mathrm{SAPni} = \quad \alpha \cdot (\mathrm{TPcl@TPW\_M\text{-}curve.max})$$

$$+ \beta \cdot (\mathrm{TPcl@TPW\_M\text{-}curve'.max}), \qquad (1)$$

wherein the parameters $\alpha$ and $\beta$ can be predetermined by calibration, particularly based on a linear regression to very accurately, possibly non-invasively determined reference values of systolic arterial blood pressure, such as by using the least squares method. Exemplary values for $\alpha$ and $\beta$, which have been determined during calibration using an exemplary dataset, are $\alpha$ = 1.43 and $\beta$ = -0.25.

Preferentially the processor 3 is configured to provide a function which receives as input the at least one feature of a respective pressure pulse and which outputs a respective blood pressure determination value TPWP_M which forms, together with the blood pressure determination values determined for the other pressure pulses, the blood pressure determination curve TPW_M-curve. The function has at least one parameter which can be determined by calibration, wherein by invasive means reference blood pressure values are determined very accurately and the at least one parameter is determined such that the apparatus yields the very accurately invasively measured blood pressure values with high statistical precision and accuracy.

According to above equation (1), once the parameters $\alpha$ and $\beta$ have been predetermined based on the reference measurements, also the systolic arterial blood pressure (SAPni) can be regarded as being determined by a fixed function of the two positions TPW_M-curve.max and TPW_M-curve'.max, wherein the linear dependence of the function on the two positions is expressed by the predetermined parameters $\alpha$ and $\beta$. Hence, the processor 3 is effectively configured to provide a first function for determining the blood pressure determination values TPWP M from the pressure pulses 9 in order to form the blood pressure determination curve TPW_M-curve, and to provide a second function corresponding to equation (1) for determining the blood pressure based on TPW_M-curve and its transformation TPW_M-curve'.

In a preferred embodiment the processor 3 is configured to form the blood pressure determination curve TPW_M-curve based on blood pressure determination values TPWP M which depend only on the feature being the difference TPP between the feature determination pulse's maximum systolic pressure TPsys and the feature determination pulse's pressure at the end-diastolic point TPdia. Thus, in an embodiment the function, which is used for determining the blood pressure determination values TPWP_M, i.e. the first function, depends on TPP, but not on any other features of the pressure pulses. In this case, for instance, the blood pressure determination values TPWP M can be determined in accordance with the following equation:

$$TPWP\_M = c \cdot TPP^{exp1}, \qquad (2)$$

wherein c and exp1 are predetermined constants. For instance, c and exp1 can be predetermined by calibration. However, it has been found that they can also both be chosen to be equal to one, i.e. c =1=exp1, such that TPWP_M = TPP.

Moreover, in a preferred embodiment the processor 3 is configured to form the blood pressure determination curve TPW_M-curve based on blood pressure determination values TPWP_M which depend only on a) the feature being the difference TPP between the feature determination pulse's maximum systolic pressure TPsys and the feature determination pulse's pressure at the end-diastolic point TPdia and b) the feature being the area TPA+.top50 enclosed by the upper part of the feature determination pulse. Thus, in an embodiment the function, which is used for determining the blood pressure determination values TPWP M, which was referred to as the first function above, depends on TPP and TPA+.top50, but not on further features of the pressure pulses. In this case, for instance, the blood pressure determination values TPWP M can be determined in accordance with the following equation:

$$TPWP\_M = c \cdot TPA+.top50^{exp2} \cdot TPP^{exp1}, \qquad (3)$$

wherein c, exp1 and exp2 are predetermined constants. For instance, c can again be equal to one, i.e. c = 1, wherein exp1 and exp2 can be predetermined by calibration for equation (3). However, it is also possible that the constant c is determined by calibration. In a preferred embodiment, c and exp 1 are both equal to one (c=1, exp1=1) and exp2 is equal to 0.7 (exp2=0.7) in equation (3).

The processor 3 preferentially is further configured to determine the end measurement time point 32, at or after which the measurement time period is stopped, based on the at least one feature determined for the plurality of pressure pulses 9. In particular, the processor 3 can be configured to determine, for each pressure pulse, an end determination value TPWP_E based on the at least one feature which has been determined for the respective pressure pulse.

Preferentially the processor is configured to also provide a function, i.e. a further function, which receives as input the at least one feature of a respective pressure pulse and which outputs a respective end determination value TPWP E which forms, together with the end determination values determined for the other pressure pulses, the end determination curve. Also this function has at least one parameter which can be determined by calibration. For instance, the end determination values TPWP E can be determined in accordance with

$$TPWP\_E = c \cdot TPP^{exp3}, \qquad (4)$$

or in accordance with

$$TPWP\_E = c \cdot TPA + .top50^{exp4} \cdot TPP^{exp3}, \qquad (5)$$

wherein c, exp3 and exp4 are predetermined constants for equations (4) and (5), respectively. For instance, c can be equal to one, i.e. c =1, while exp3, and possibly exp4, can be predetermined by calibration to fix equations (4) and (5), respectively. In an embodiment, it is possible for any of equations (4) and (5) that also the constant c is determined by calibration and particularly not 1.

It should be noted that constants like c, exp1, exp2 and exp3, which appear in more than one equation as is the case above, can generally have the same or different values in the respective different equations. Hence, for instance, these constants could also be referred to with an additional index associated with the respective equation, which is however omitted herein. Nevertheless, is should be understood that, for instance, for fixing above equations (2) to (5), six exponent values are needed.

It is also possible that the end determination values TPWP_E are determined as follows.

In particular, the processor 3 can be configured to multiply a) the determined pulse area TPA.norm to the power of a predetermined seventh exponent (exp7) with b) the determined difference TPP to the power of a predetermined eighth exponent (exp8). Thus, an end determination value TPWP_E can be calculated in accordance with the equation $TPWP\_E=TPA.norm^{exp7} \cdot TPP^{exp8}$, with predetermined $exp7 \neq 0$, $exp8 \neq 0$.

The processor 3 can also be configured to determine an end determination value TPWP E by dividing a) the determined pulse area TPA.norm to the power of a predetermined ninth exponent (exp9) by b) the determined pulse duration t(pulse) to the power of a predetermined tenth exponent (exp10) and multiplying the resulting quotient with c) the determined difference TPP to the power of a predetermined eleventh exponent (exp11). This can be expressed as $TPWP\_E=TPA.norm^{exp9}/ t(Pulse)^{exp10} \cdot TPP^{exp11}$, with predetermined $exp9 \neq 0$, $exp10 \neq 0$, $exp11 \neq 0$.

The processor 3 can also be configured to determine an end determination value for a respective pressure pulse based on multiplying the calculation result as obtained by using, for instance, one of the equations $TPWP\_E=TPA.norm^{exp7} \cdot TPP^{exp8}$ and $TPWP\_E=TPA.norm^{exp9}/ t(Pulse)^{exp10}TPP^{exp11}$ discussed in the two previous paragraphs, with the width at half maximum (W50) to the power of a further predetermined exponent. For instance, if the former of the two equations is multiplied by the width at half-maximum W50, the equation $TPWP\_E=TPA.norm^{exp12} /t(Pulse)^{exp13} \cdot TPP^{exp14} \cdot W50^{exp15}$ results, with predetermined $exp6 \neq 0$, $exp7 \neq 0$, $exp8 \neq 0$, $exp9 \neq 0$.

Thus, in this embodiment based on at least TPP and TPA.norm for a respective pulse curve a TPWP E can be calculated that reflects a characteristic value for the respective pulse curve by combining and weighting the amplitude and area parameters, wherein any of the TPWP E can be extended by multiplying with W50 to the power of a respective exponent.

The exponents for the different equations are predetermined, as mentioned above, by calibration. For instance, in a preferred embodiment equation $TPWP\_E=TPA.norm^{exp9}/ t(Pulse)^{exp10} \cdot TP^{exp11}$ is used with the exponents exp9 = 1.1, exp10 = 0.4, exp11 = 0.5.

The processor 3 is preferentially configured to determine the end determination curve TPW_E-curve such that it fulfills one of the following conditions: a) a maximum of the end determination curve (TPW_E-curve.max) occurs temporally before the maximum of the blood pressure determination curve (TPW_M-curve.max), b) a maximum of the end determination curve (TPW_E-curve.max) occurs temporally at or after a maximum of the blood pressure determination curve (TPW_M-curve.max) and the decrease of the end determination curve TPW_E-curve after its maximum is steeper than the decrease of the blood pressure determination curve TPW_M-curve after its maximum, and c) the end determination curve TPW_E-curve is identical to the blood pressure determination curve TPW_M-curve. Thus, the parameters of the functions are preferentially predefined such that a) TPW_E-curve.max occurs temporally before TPW_M-curve.max or b) TPW E-curve.max occurs at the time of TPW_M-curve.max or temporally after, but TPW_E-curve has a negative derivative with a larger absolute derivative value than the absolute derivative value of the negative derivative of the blood pressure determination curve TPW_M-curve after its maximum or c) TPW E-curve can be identical to TPW_M-curve, i.e. the end determination curve and the blood pressure determination curve can be identical.

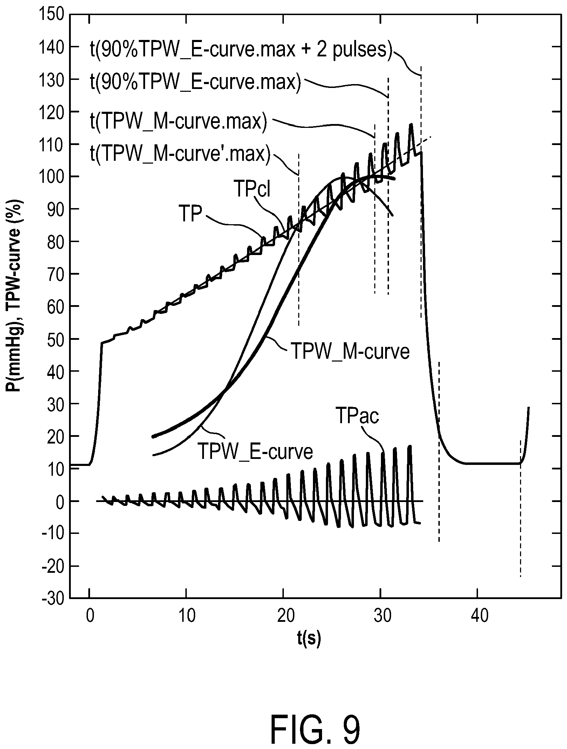

In Fig. 9 a blood pressure determination curve TPW_M-curve and an end determination curve TPW_E-curve are exemplarily shown, wherein these curves have been normalized such that the respective maximum corresponds to 100 percent.

It is desirable to terminate each blood pressure measurement as soon as possible to minimize stress for the subject, i.e. for the subject. The blood pressure measurement can in this case be terminated as soon as the maximum of the blood pressure determination curve TPW_M-curve has been formed and hence can be completely determined, because in this case the maximum of the blood pressure determination curve TPW_M-curve is used for the noninvasive blood pressure determination. It is noted that, if only the maximum of the derivative TPW_M-curve' of the blood pressure determination curve were used, the blood pressure measurement could be terminated already as soon as this maximum has been formed. Thus, during the measurement time period it is checked when an end criterion is reached, which defines the end measurement time point, wherein for determining when this end criterion is reached and hence for determining the end measurement time point preferentially the end determination curve TPW_E-curve and the blood pressure determination curve TPW_M-curve are used. For instance, when the end determination curve TPW_E-curve has formed its maximum, it can be checked with each newly measured TP pulse curve, i.e. with each newly measured TP pulse, if a) the end determination curve TPW_E-curve has decreased below a percentage of the maximum of the end determination curve TPW_E-curve, wherein this percentage is preferentially within a range from 40 percent to 95 percent, and b) the blood pressure determination curve TPW_M-curve has formed its maximum, i.e. has reached or passed its maximum. The processor 3 can be configured to determine the end measurement time point 32 as the time point at which both conditions a) and b) are met.

In order to determine whether the blood pressure determination curve TPW_M-curve has reached or passed its maximum safely, it can be detected whether the blood pressure determination curve TPW_M-curve has fallen after its maximum.

In an embodiment any of the blood pressure determination curve TPW_M-curve and its transformed version TPW_M-curve' can be a smoothed curve such that any maxima of the two curves are also smoothed. The smoothing procedure used for smoothing the blood pressure determination curve TPW_M-curve can include, for instance, filtering and/or fitting, wherein the transformed blood pressure determination curve TPW_M-curve' may be smooth already by virtue of the blood pressure determination curve itself and the transformation applied to it being smooth. Taking a derivative or applying a smooth function, for instance, are smooth transformations. Further, several pressure pulses can be considered, after the respective smoothed maximum, to detect the respective smoothed maximum unambiguously, wherein the number of these several pulses can be predetermined or can depend on the heart rate of the subject. For instance, this number can increase with increasing heart rate. In an embodiment this number is 3 for a heart rate of 60/min and 10 for a heart rate of 200/min.

For smoothing the blood pressure determination curve TPW_M-curve a moving average filter, particularly a variable moving average filter, can be used that is applied on the blood pressure determination values TPWP_M determined for the pressure pulses. The window used for the averaging can be fixed or variable, wherein in the latter case it preferentially has a maximum duration of, for instance, 8 s. The use of this filter causes a filter delay, wherein the minimum filter delay is as long as the added up durations of the several pulses. Having a filter delay with a length of few pulses suffices to safely determine a falling smoothed blood pressure determination curve TPW_M-curve and therefore a maximum in the blood pressure determination curve TPW_M-curve. That means as soon as a decrease in the smoothed blood pressure determination TPW_M-curve is detected, the measurement time period will be terminated without needing to collect more pressure pulses. This is possible because of the, in this example, smoothing of the blood pressure determination curve TPW_M-curve with relatively large variable moving average filtering windows of, for instance, at least three TP pressure pulses and maximal 8 s twice (2 x 8 s filter) that removes disturbing heart-lung interaction in estimated 95 percent of measurements. These smoothing filter windows can be made adaptive to the respiratory rate respectively ventilation rate, thereby allowing to improve the elimination of unwanted TPW_M-curve disturbances caused by heart-lung interaction.

In Fig. 9 both of the above mentioned conditions a) and b) are met, when the end determination curve TPW_E-curve has decreased below 90 percent of the maximum of the end determination curve TPW_E-curve. Due to the filter delay the measurement is terminated a few tissue pressure pulses after both and conditions a) and b) have been met.

The continuous TPW_E-curve and the continuous TPW_M-curve shown in Fig. 9 have been obtained by filtering the values TPWP E and TPWP M, respectively, which had been determined for the different pressure pulses. This filtering, which leads to the filter delay, will be explained further below. In Fig. 9 the filter delay results in two additional pressure pulses such that the fast deflation of the cuff 6 starts two pressure pulses after both conditions a) and b) had been met.

An end determination curve TPW_E-curve having a maximum temporally occurring before the maximum of the blood pressure determination curve TPW_M-curve by some seconds allows for a shortening of the measurement time period, i.e. allows for a shortening of the slow inflation time and reduced amount of pressure applied to the subject. After reaching t(a_end), i.e. after reaching the time point at which the end determination curve TPW_E-curve has decreased below a predefined percentage of the maximum of the TPW_E-curve, the largest maximum of the blood pressure determination curve TPW_M-curve found so far, if the TPW_M-curve has several maxima, is the absolute maximum of the blood pressure determination curve TPW_M-curve (TPW M-curve.max), if the blood pressure determination curve TPW_M-curve is decreasing at the time t(a_end). Otherwise, in an embodiment, a measurement will be continued until the next maximum of the TPW_M-curve and the maximum of the blood pressure determination curve (TPW_M-curve.max) will be determined afterwards.

Without using the end determination curve TPW_E-curve with a maximum temporally occurring before the maximum of the blood pressure determination curve TPW_M-curve, the slow inflation would need to continue until the blood pressure determination curve TPW_M-curve has fallen below a certain percentage of its maximum. In average by using the end determination curve TPW E-curve with a maximum occurring temporally before the maximum of the blood pressure determination curve TPW_M-curve, a blood pressure measurement can be terminated after about 20 to 60 seconds at a TPcl within a range of 70 to 95 percent of systolic arterial pressure (SAP). This is significantly below the end pressure level of a conventional oscillometric niBP measurement. There the blood pressure measurement is terminated when the clamping pressure has reached about SAP + 20 mmHg. When using the apparatus 1 as described above with reference to Fig. 1 and when the measurement is terminated, the tissue pressure TP drops as fast as possible to Patt remaining there for some time, preferably for about 20 percent of the inflation-deflation time period 40 indicated in Fig. 4, before another measurement is started.

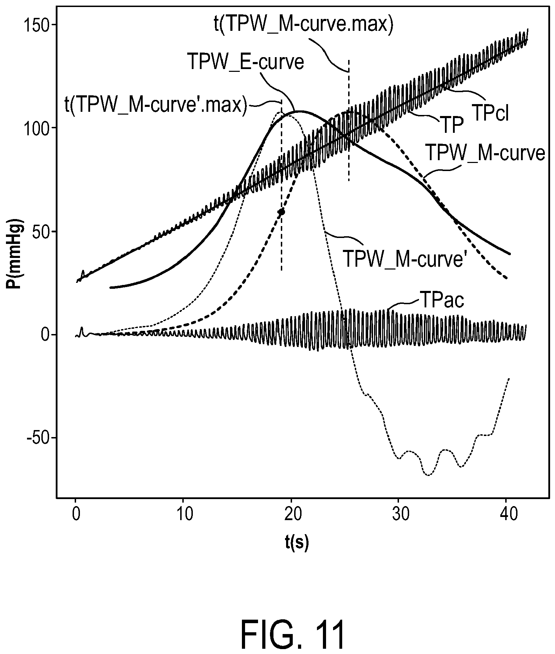

The processor 3 is configured to determine the blood pressure based on the transformed blood pressure determination curve TPW_M-curve', especially based on the position of the maximum of the derivative of the blood pressure determination curve TPW_M-curve, and in this case further based on the blood pressure determination curve TPW_M-curve itself, especially based on the position of the maximum of the blood pressure determination curve TPW_M-curve itself. The processor 3 can be adapted to perform this determination of the blood pressure, which is a noninvasive blood pressure, as described above with respect to equation (1). While being determined by the processor 3, the curve TPW_M-curve' is not included in Fig. 9. In case the transformation from which it results is chosen to be a derivative, however, TPW_M-curve' can be deduced from the slope of TPW_M-curve in Fig. 9. An example in which TPW_M-curve and TPW M-curve' have been included in a single diagram is illustrated by Fig. 11, which will be described further below.

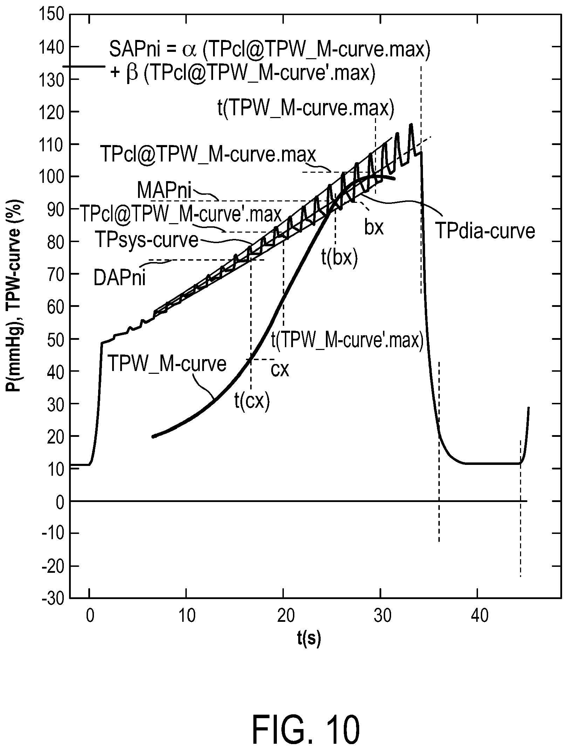

In an embodiment the processor 3 is configured to determine the TPcl value at the time point t(TPW_M-curve'.max) at which the derivative of the blood pressure determination curve TPW_M-curve' has its maximum (TPW_M-curve'.max) and the TPcl value at the time point t(TPW _M-curve.max) at which the blood pressure determination curve TPW_M-curve has its maximum (TPW M-curve.max), wherein in Fig. 10 the TPcl values at the respective times of the maxima are denoted as "TPcl@TPW_M-curve'.max" and "TPcl@TPW_M-curve.max", respectively. While also in Fig. 10 the curve TPW_M-curve' is not shown, from the slope of TPW_M-curve, which itself has its maximum at t(TPW_M-curve.max), the time point t(TPW_M-curve'.max) at which the derivative TPW_M-curve' of TPW_M-curve has its maximum can be deduced. The time points t(TPW_M-curve.max) and t(TPW_M-curve'.max) are also indicated in Fig. 9.

The processor 3 is further configured to determine a lower envelope of the tissue pressure TP by applying a filter to the end-diastolic points of the tissue pressure TP. The filter can be the same as the filter used for determining, for instance, the blood pressure determination curve TPW_M-curve. The resulting curve is named "TPdia-curve" in Fig. 10. Moreover, the processor 3 can be configured to determine an upper envelope of the tissue pressure TP by applying a filter to the systolic maxima of the tissue pressure TP. In addition, this filter can be similar to the filter used for, for instance, generating the blood pressure determination curve TPW_M-curve. This curve is named "TPsys-curve" in Fig. 10.

The processor 3 can then be configured to determine the systolic arterial pressure value as a predetermined linear combination of TPcl@TPW_M-curve'.max and TPcl@TPW_M-curve.max, i.e. a linear combination with predetermined coefficients $\alpha$ and $\beta$ corresponding to a calibrated function as given by above equation (1). The predetermined coefficients $\alpha$ and $\beta$ are preferentially such that the linear combination results in a systolic arterial pressure value expressible as a percentage (TPcl.s%) of TPcl@TPW_M-curve.max, wherein the percentage preferentially lies within a range from 100 to 150 percent, further preferred from 110 to 150 percent. In Fig. 10 the noninvasive systolic arterial pressure is named "SAPni", wherein above equation (1) is shown again. In Fig. 10 the noninvasive systolic arterial pressure SAPni is about 134 mmHg.

The predetermined coefficients $\alpha$ and $\beta$ can also be such that the noninvasive systolic arterial pressure SAPni is a predetermined percentage (TPsys.s%) of the TPsys curve at the temporal position at which the blood pressure determination curve TPW_M-curve has its maximum. This predetermined percentage is preferentially within a range from 100 to 140 percent.

The processor 3 can also be configured to determine the noninvasive mean arterial pressure (MAPni) based on the transformed TPW_M-curve, particularly its derivative. For instance, an equation like above equation (1) can again be used, i.e.

$$\text{MAPni} = \quad \alpha' \cdot (\text{TPcl@TPW\_M-curve.max})$$
$$+ \beta' \cdot (\text{TPcl@TPW\_M-curve'.max}) \qquad , \quad (6)$$

with new predetermined, i.e. correspondingly calibrated coefficients $\alpha'$ and $\beta'$. The predetermined coefficients $\alpha'$ and $\beta'$ can be such that the determined noninvasive mean arterial pressure (MAPni) corresponds to the TPcl value or alternatively the value of the TPdia-curve or alternatively the value of the TPsys-curve at a time point t(bx) being the time point at which the blood pressure determination curve TPW_M-curve has a value bx representing a predetermined percentage relative to its maximum. The predetermined coefficients $\alpha'$ and $\beta'$ can be such that the noninvasive mean arterial pressure MAPni is a predetermined percentage (TPcl.m%) of TPcl at TPW_M-curve.max, wherein the predetermined percentage is preferentially within a range from 80 to 110 percent. Exemplary values for $\alpha'$ and $\beta'$, which have been determined during calibration using an exemplary dataset, are $\alpha' = 0.44$ and $\beta' = 0.46$.

The processor 3 can also be configured to determine the noninvasive diastolic arterial pressure DAPni based on the transformed TPW_M-curve, particularly its derivative. For instance, an equation like above equations (1) and (6) can again be used, i.e.

$$DAPni = \quad \alpha'' \cdot (TPcl@TPW\_M\text{-}curve.max)$$
$$+ \beta'' \cdot (TPcl@TPW\_M\text{-}curve'.max) \qquad , \qquad (7)$$

with new predetermined, i.e. correspondingly calibrated coefficients $\alpha''$ and $\beta''$. The predetermined coefficients $\alpha''$ and $\beta''$ can be such that the determined noninvasive diastolic arterial pressure (DAPni) corresponds to the value of the TPdia-curve or alternatively of the TPcl curve at a time point t(cx) being the time point at which the blood pressure determination curve TPW_M-curve has a value cx representing a predetermined percentage of its maximum. The predetermined coefficients $\alpha''$ and $\beta''$ can be such that DAPni is a predefined percentage (TPcl.d%) of TPcl@TPW_M-curve.max, wherein this predetermined percentage is preferentially within a range from 60 to 80 percent. Exemplary values for $\alpha''$ and $\beta''$, which have been determined during calibration using an exemplary dataset, are $\alpha'' = -0.08$ and $\beta'' = 0.88$.

As explained above, the parameters of the functions used for determining the end determination curve TPW_E-curve and for determining the blood pressure determination curve TPW M-curve are determined by calibration. Thus, these parameters are predetermined such that, during a calibration phase, deviations between very accurately measured invasive blood pressure values and the blood pressure values obtained by the apparatus 1 are minimized and the time needed for a blood pressure measurement is relatively low. Preferentially, thereby the one or several parameters of the function used for determining the blood pressure determination curve TPW_M-curve, i.e. the function referred to as the first function further above, which might be regarded as being a first set of one or several parameters, is chosen in a way to achieve a good balance between high accuracy noninvasive blood pressure on one hand and a low TP level at the end of the measurement and therefore a low measurement time on the other hand. If the one or several parameters were chosen such that they create a TPW_M-curve with a relatively early maximum, the TP level at the end of the measurement and the measurement time would be relatively low. However, the accuracy and position of the finally determined noninvasive blood pressure values is then also reduced. Thus, the first set of one or several parameters is preferentially chosen such that a desired balance between measurement time and accuracy of the noninvasive blood pressure values is achieved.

The one or several parameters of the function used for determining the end determination curve TPW_E-curve, i.e. the function referred to as a further function further above, which might be regarded as forming a further set of one or several parameters, are preferentially chosen such that the maximum of the end determination curve TPW_E-curve is relatively early. In an embodiment the first set of one or several parameters and the second set of one or several parameters can also be the same such that the end determination curve TPW_E-curve and the blood pressure determination curve TPW M-curve can also be the same.

The calibration leading to the predetermined values of the coefficients $\alpha$, $\beta$, $\alpha'$, $\beta'$, $\alpha''$ and $\beta''$ in the functions describing the systolic arterial blood pressure (SAPni), the mean arterial blood pressure (SAPni) and the diastolic arterial blood pressure (SAPni) in terms of the positions of the maxima of the blood pressure determination curve and its derivative, i.e. the functions of which each could be referred to as a second function in the terminology used further above, preferentially corresponds to an evaluation of a calibration set consisting of measurement pairs of simultaneously recorded invasive blood pressure values and noninvasive blood pressure values from an adequate number of individuals in different hemodynamic conditions. The noninvasive blood pressure values are determined as described above by the apparatus 1 and the parameters like the coefficients $\alpha$, $\beta$, $\alpha'$, $\beta'$, $\alpha''$ and $\beta''$ can be optimized such that deviations between invasive blood pressure values and noninvasive blood pressure values are minimized.

Fig. 11 illustrates the curves TP, TPcl, TPac, TPW_M-curve and TPW_E-curve according to a further example. Hence, these curves are determined like their correspondents illustrated in Figs. 9 and 10, but based on a further measurement, resulting in a different measured pressure signal TP. Unlike Figs. 9 and 10, Fig. 11 also shows the derivative TPW_M-curve' of TPW_M-curve, which is used for determining the blood pressure. Also the time points t(TPW_M-curve.max) and t(TPW_M-curve'.max) at which TPW_M-curve and TPW_M-curve' have their respective maximum are again indicated in Fig. 11. Correspondingly, TPcl@TPW_M-curve.max and TPcl@TPW_M-curve'.max can be identified also in this example, such that the blood pressure can again be determined in accordance with any of above discussed equations (1), (6) and (7).

The processor 3 can be adapted to estimate a kind of blood pressure value based on two other already measured kinds of blood pressure values. In particular, one of the blood pressure values MAPni, SAPni and DAPni can be estimated based on the other of these blood pressure values. This may be done in accordance with following equations:

$$SAPni = c1 \cdot MAPni + c2 \cdot (MAPni - DAPni) - c3 \text{ mmHg} \qquad (8)$$

with $c1 = (0.2 .. 0.7)$, $c2 = (2 .. 6)$, $c3 = (-5 .. 5)$,

$$MAPni = c4 \cdot DAPni + c5 \cdot (SAPni - DAPni) - c6 \text{ mmHg} \qquad (9)$$

with $c4 = (0.8 .. 1.3)$, $c5 = (0.25 .. 0.5)$, $c6 = (-5 .. 5)$,

$$DAPni = c7 \cdot MAPni - c8 \cdot (SAPni - MAPni) - c9 \text{ mmHg} \qquad (10)$$

with $c7 = (0.6 .. 1.1)$, $c8 = (0.15 .. 0.4)$, $c9 = (-5 .. 5)$.

The coefficients and constants of equations (8), (9) and (10) are predetermined by calibration based on a statistical evaluation of an as large as possible and adequately widely spread set of clinical invasive blood pressure data. Thus, very accurate invasive blood pressure values SAPi (invasive systolic arterial pressure), MAPi (invasive mean arterial pressure) and DAPi (invasive diastolic arterial pressure) are used, wherein, given these very accurate invasive blood pressure values, the coefficients and constants of the equations (8), (9) and (10) are modified such that these equations are valid.

The blood pressure measurement is intended to be used in a sequence of measurements in rapid succession to allow for effective semi-continuous blood pressure monitoring such that the stress for the monitored individual, i.e. for the monitored subject, is minimized. This sequence of fast pressure measurements can also be regarded as being a noninvasive fast mode cycle (FMC) measurement of the blood pressure.

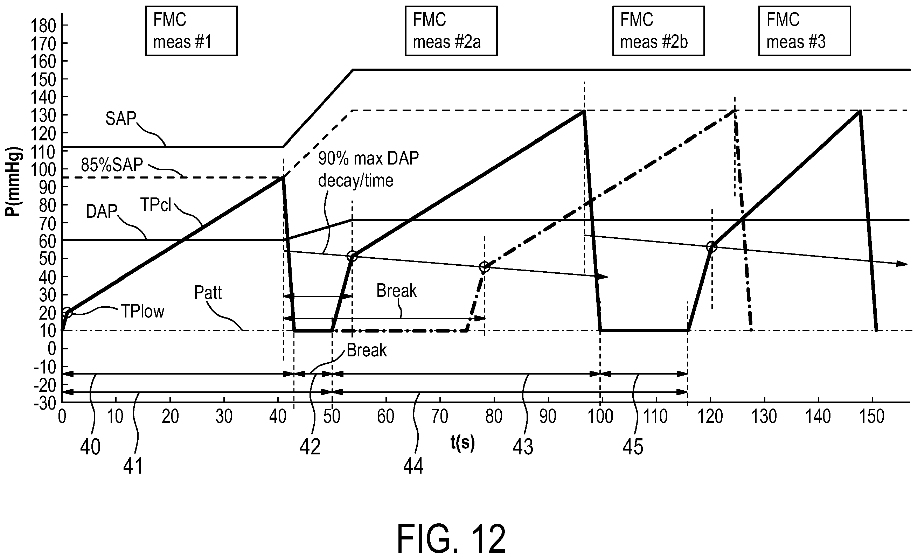

In an embodiment the initial blood pressure measurement has no information about a previous diastolic arterial pressure (DAP) and is therefore starting the slow inflation, i.e. the measurement time period, at a predetermined tissue pressure TPlow within a range from 15 to 30 mmHg. Moreover, in this measurement time period the inflation rate is set to a medium value. The inflation rate can be defined as the rate with which TPcl increases over time, wherein this inflation rate can be chosen such that it has a value of, for instance, 1.9 mmHg/s. This would correspond to a measurement of "normal" blood pressure values. At the end of the deflation phase, when TPcl has reached Patt, the blood pressure measurement preferentially has a break of at least 2 s, in order to allow reperfusion of the body part of the subject encased by the shell 4. This break time 42 has preferentially a length within a range from more than 0 to about 50 percent of the preceding inflation-deflation time period 40 resulting in cycle 41. The following blood pressure measurements will use the information of the diastolic arterial pressure (DAP) determined in the respective preceding measurement (DAPprev) to start the slow inflation, i.e. the measurement time period, with a higher TPlow as shown in Fig. 12.

The TPlow value should not be above 90 percent of the diastolic arterial pressure to ensure that all pulses necessary for the calculations of blood pressure are recorded. Furthermore, as explained above, the blood pressure can change in time intervals (t_inter) between a start of deflation and a start of a next slow inflation. Hence, preferentially a function to model the blood pressure decrease depending on the time intervals t inter is applied. It has been observed in clinical data from high-risk surgery that the diastolic arterial pressure can decrease by more than 28 percent within one minute. Thus, TPlow could be calculated by using a linear function like the following function:

$$TPlow = 90\%DAPprev - 28\%DAPprev \cdot t\_inter / 60 \text{ s}, \qquad (11)$$

wherein "90%DAPprev" and "28%DAPprev" denote 90 percent and 28 percent, respectively, of the previously measured diastolic arterial pressure DAPprev.

TPlow can be determined by using a linear function, which depends on the previous time interval between the start of deflation and the start of the next slow inflation, wherein the linear function has a negative slope such that TPlow decreases with time. The slope of the linear function and also the positive constant of the linear function can be predetermined by calibration. In equation (11) the positive constant is 90%DAPprev and the negative slope is -28%DAPprev. The positive constant and the negative slope could also have other values. For instance, the negative slope could be -30%DAPprev.

In Fig. 12 it can be seen how TPlow is adapted depending on t_inter in accordance with equation (11). It can especially be seen that the measurements #2a and #2b have different TPlow values, wherein the TPlow value for the measurement #2b is smaller than the TPlow value for the measurement #2a, because for the measurement #2b the time interval t_inter is larger than for the measurement #2a. In Fig. 12 it can also be seen that the inflation rate for the measurement #3 is increased due to an increase of PP before the measurement #2a. Moreover, for the measurement #3 TPlow is larger in comparison to the previous measurements, because the diastolic arterial pressure DAP has increased before the measurement #2a. It should be noted that in Fig. 12 the interval 44 is the cycle period for the second measurement #2a, the interval 43 is the inflation-deflation period for the second measurement #2a and the interval 45 is the break time for the second measurement #2a. Moreover, it should be noted that the third measurement #3 refers to the second measurement #2a and that the further second measurement #2b is just shown for illustrating an alternative to the second measurement #2a.

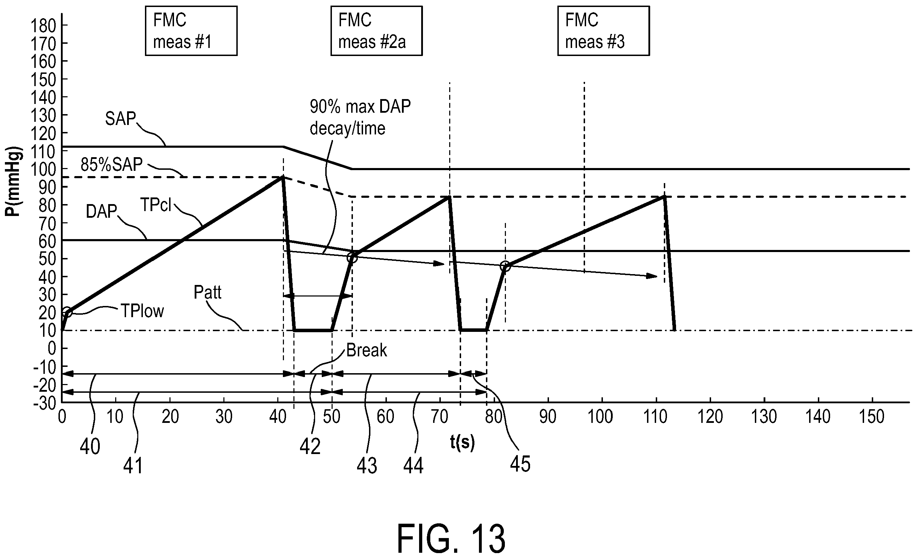

Fig. 13 shows a further example for adapting the TPlow value depending on the time interval t_inter in accordance with the equation (11). In this example the inflation rate in the measurement time period is decreased for the measurement #3 in comparison to the previous measurement due to a PP decrease before the measurement #2a and a lower TPlow value due to a decrease of DAP before the measurement #2a.

As explained above, for determining the end determination curve TPW_E-curve, the blood pressure determination curve TPW_M-curve and the envelope curves TPsys-curve and TPdia-curve a filter is used. Moreover, also for determining TPcl based on TP a filter is used. As the filter preferentially a low pass filter is used. The low pass filter can be, for instance, a cascaded moving average filter with a varying window length of up to 8 s, wherein the window length can be shorter at the beginning and at the end of the filtering, in order to a minimize filter settling time. For instance, the filtering can include averaging within a moving window containing signals of at least three TP pressure pulse curves and maximal 8 s twice (2 x 8 s filter). Optionally, a signal padding can be applied before the beginning and/or after the end of the signal for fully filling the filter windows before filtering. For instance, padding with the first pressure pulse's value before the beginning and the last pressure pulse's value after the end can be applied.

Due to the filter delay TPcl is extrapolated from its previous values to be available for the last part of the measured signal. The filter can be applied to, for instance, extract TPcl from TP, form a smooth TPW_E-curve and a smooth TPW_M-curve without variation caused by blood pressure pulsation and to create an envelope function over systolic peaks (TPsys-curve) and over end-diastolic minima (TPdia-curve) of TP.

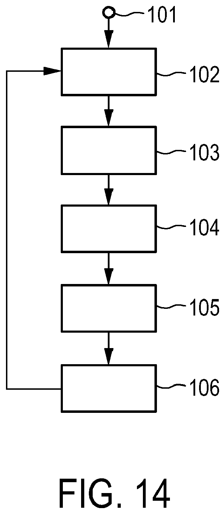

In the following an embodiment of a method for determining blood pressure will be exemplary described with reference to a flowchart shown in Fig. 14.

After initializing and starting the apparatus in step 101, in step 102 TPlow is determined. For a first measurement it can be a value from 15 mmHg to 30 mmHg. If blood pressure measurements have already been carried out, a previous diastolic arterial blood pressure value could be used, for instance in accordance with equation (11), for determining TPlow.

In step 103 in the pre-measurement time period the cuff is inflated with a relatively large first inflation rate until TPlow has been reached. Then, in step 104 the measurement time period, i.e. the slow inflation period, starts, wherein during this measurement time period the blood pressure determination curve TPW_M-curve and the end determination curve TPW_E-curve are determined. Moreover, these curves are used for determining an end measurement time point, wherein, when this end measurement time point has been reached or passed, the slow inflation period is stopped and in step 105 the cuff is deflated. After deflation and a pause for allowing venous return in step 106, the method continues with step 102. Thus, the method can be carried out in a loop for continuously monitoring the blood pressure over time in several measurement cycles. While carrying out steps 102 to 105, the processor calculates the blood pressure value in parallel. The loop can be carried out until an abort criterion is fulfilled. For instance, the monitoring of the blood pressure is interrupted, if a physician has inputted a corresponding command into the apparatus via an input unit like a keyboard, a computer mouse, a touchpad, et cetera.

The apparatus allows for a noninvasive FMC measurement of blood pressure based on noninvasive high-fidelity tissue pressure TP recordings preferably recorded with a kinking-proof shell system as described in WO 2014/121945 A1. The apparatus preferentially allows for a statistical mean maximum pressure inflation of about 85 percent of the systolic arterial pressure or less. A single blood pressure measurement by using the apparatus 1 may take about 20 to 60 s depending on the height of arterial blood pressure, of pulse pressure, and inversely of heart rate, wherein the apparatus is preferentially configured to provide the noninvasive systolic arterial pressure SAPni, the noninvasive mean arterial pressure MAPni and the noninvasive diastolic arterial pressure DAPni. The measurements are preferentially executed with short breaks with an unpressurized cuff between consecutive measurements to allow venous return. These short breaks are preferentially within a range from 5 to 10 s. During the measurement arterial perfusion of the part encased by the shell like an upper arm or a wrist of a subject is preferentially never completely interrupted, because clamping pressure preferentially permanently stays below SAP. The measurement is therefore less stressful to the subject compared to conventional oscillometric measurements.

The apparatus 1 described above with reference to, for instance, Fig. 1 provides a reliable method for a fast determination of blood pressure with clamping pressures not exceeding SAP. Due to a relatively short measurement time and a relatively low maximal cuff pressure, this blood pressure measurement is very convenient for the individual and the likelihood of potential complications such as thrombophlebitis, pain, ecchymosis, limp edema, peripheral neuropathy, et cetera can be reduced.

In an embodiment TPlow can be at DAP or higher. TPlow defines the tissue pressure at the beginning of the measurement time period, i.e. at the beginning of the slow inflation period, wherein the slow inflation may be terminated when a certain end criterion is reached. End criteria have been described above.

Although in above described embodiments the features are determined by using the TPac pulses, the features can also be determined by using directly the measured pressure pulses, i.e. the TP pulses. It is also possible to process the TP pulses in another way, i.e. not by subtracting the mean TP value for determining the TPac pulses. For instance, for the respective measured pressure pulse the pressure values at t.start and t.stop can be connected by a straight line and this straight line can be subtracted from the respective measured pressure pulse, in order to determine a feature determination pulse.

Although in above described embodiments the TPW_M-curve and the TPW_E-curve have been determined, it is also possible that the TPW_M-curve is determined and not the TPW_E-curve, wherein a transformation of the TPW_M-curve, and possibly also the TPW_M-curve itself, is used for determining the blood pressure.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Calculations like the determination of an end measurement time point, of features of pulses, of certain curves and their transformations, of blood pressure values, et cetera performed by one or several units or devices can be performed by any other number of units or devices. The calculations and determinations and/or the control of the apparatus in accordance with the method can be implemented as program code means of a computer and/or as dedicated hardware.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope. In particular, the letters like TPP, TPA, et cetera used in brackets in the claims as reference signs should not be construed as limiting the scope. For instance, also other features than the features, which are represented by the reference signs, can be used in accordance with the claims.

An apparatus for determining blood pressure of a subject is presented. The apparatus comprises a pressure signal providing unit configured to provide a measured pressure signal of a subject over a time, wherein the pressure signal is indicative of blood pulsations and comprises a plurality of pressure pulses. The apparatus further comprises a processor that is configured to determine, based on the plurality of pressure pulses, a blood pressure determination curve indicative for a dependence of the pressure pulses on the applied pressure, to apply a transformation to the blood pressure determination curve and to determine the blood pressure based on a result of the transformation. This allows to determine the blood pressure more accurately.

**Claims**

1. An apparatus (1) for determining blood pressure of a subject, the apparatus comprising:

   - a pressure signal providing unit configured to provide a measured pressure signal (TP) of a subject over a time, wherein the pressure signal (TP) has been measured by using a measurement device comprising a) a shell (4) configured to encase a part (5) of the subject, through which blood flows, b) a pressure applicator (6, 8) configured to apply, from outside the shell (4), pressure to the shell (4) and thereby to the encased part (5) of the subject, and c) a pressure sensor (7) configured to measure the pressure signal (TP) on the skin of the encased part (5) of the subject, wherein the pressure applicator (6, 8) increases or decreases the applied pressure while the pressure signal (TP) is measured, and wherein the measured pressure signal (TP) is indicative of blood pulsations and comprises a plurality of pressure pulses (9), and
   - a processor (3) configured to determine a blood pressure determination curve (TPW_M-curve) based on the plurality of pressure pulses (9), wherein the blood pressure determination curve (TPW _M-curve) is indicative for a dependence of the pressure pulses (9) on the applied pressure, and wherein the processor is further configured to apply a transformation to the blood pressure determination curve (TPW M-curve) and to determine the blood pressure based on a result of the transformation.

2. The apparatus (1) as defined by claim 1, wherein the transformation applied to the blood pressure determination curve (TPW_M-curve) refers to taking a derivative (TPW_M-curve') of the blood pressure determination curve

(TPW_M-curve), such that the processor (3) is configured to determine the blood pressure based on the derivative (TPW M-curve') of the blood pressure determination curve (TPW M-curve).

3. The apparatus (1) as defined by claim 2, wherein the processor (3) is configured to determine a position of a maximum of the derivative (TPW M-curve') of the blood pressure determination curve (TPW M-curve) and to determine the blood pressure based on the determined position and the measured pressure (TP).

4. The apparatus (1) as defined by claim 3, wherein the processor (3) is configured to determine the blood pressure based on the mean (TPcl) of the measured pressure (TP) at the determined position of the maximum.

5. The apparatus (1) as defined by claim 3 or 4, wherein the processor (3) is further configured to determine a position of a maximum of the blood pressure determination curve (TPW M-curve) and to determine the blood pressure based further on this further determined position and the measured pressure (TP).

6. The apparatus (1) as defined by claim 5, wherein the processor (3) is configured to determine the blood pressure based further on the mean (TPcl) of the measured pressure (TP) at the determined further position.

7. The apparatus (1) as defined by claims 4 to 6, wherein the processor (3) is configured to determine the blood pressure as a function of a) the mean (TPcl) of the measured pressure (TP) at the determined position of the maximum of the derivative (TPW M-curve') of the blood pressure determination curve (TPW M-curve) and b) the mean (TPcl) of the measured pressure (TP) at the determined position of the maximum of the blood pressure determination curve (TPW M-curve), wherein the dependence of the function on the mean (TPcl) of the measured pressure (TP) at the two positions is predetermined based on reference measurements.

8. The apparatus (1) as defined by claim 7, wherein the function is a linear function whose dependence on the mean (TPcl) of the measured pressure (TP) at the two positions is expressed by linear coefficients, wherein the linear coefficients are predetermined in a linear regression carried out based on the reference measurements.

9. The apparatus (1) as defined by any of the preceding claims, wherein the processor (3) is configured to determine, for a respective pressure pulse of the plurality of pressure pulses (9), at least one feature that characterizes the respective pressure pulse, to determine, for the respective pressure pulse, a blood pressure determination value (TPWP_M) based on the determined at least one feature such that for several pressure pulses, which are present at different times, several blood pressure determination values (TPWP _M) are determined, wherein the processor (3) is configured to determine the blood pressure determination curve (TPW_M-curve) such that the several blood pressure determination values (TPWP _M) determined for the several pressure pulses and hence for several times form the blood pressure determination curve (TPW_M-curve).

10. The apparatus (1) as defined by claim 9, wherein the processor (3) is configured to, in order to determine for a respective pressure pulse the at least one feature, provide a feature determination pulse (29) based on the respective pressure pulse, and determine at least one of the following features:

   - the difference (TPP) between the feature determination pulse's maximum systolic pressure (TPsys) and the feature determination pulse's pressure at the end-diastolic point (TPdia),
   - the area (TPA+.top50) enclosed by an upper part of the feature determination pulse, wherein a) the upper end of the upper part is at the feature determination pulse's maximum systolic pressure (TPsys) and b) the lower end of the upper part is between the feature determination pulse's maximum systolic pressure (TPsys) and a pressure value which corresponds to a mean (TPcl) of the measured pressure (TP),
   - the duration (t(pulse)) of the respective feature determination pulse (29),
   - the area (TPA/TPA.norm) of the respective feature determination pulse (29), and
   - the width at half maximum (W50) of the respective feature determination pulse (29).

11. The apparatus (1) as defined by claim 10, wherein the processor (3) is configured to determine the blood pressure determination values (TPWP_M) and hence form the blood pressure determination curve (TPW M-curve) only based on the feature being the difference (TPP) between the feature determination pulse's maximum systolic pressure (TPsys) and the feature determination pulse's pressure at the end-diastolic point (TPdia).

12. The apparatus (1) as defined by claim 11, wherein the processor (3) is configured to determine the blood pressure determination values (TPWP_M) and hence form the blood pressure determination curve (TPW M-curve) only based on a) the feature being the difference (TPP) between the feature determination pulse's maximum systolic pressure (TPsys) and the feature determination pulse's pressure at the end-diastolic point (TPdia) and b) the feature being the area (TPA+.top50) enclosed by the upper part of the feature determination pulse.

13. The apparatus (1) as defined by any of the preceding claims, wherein the processor (3) is configured to control the measurement device such that the applied pressure is increased in a measurement time period extending till an end measurement time point (32) and the applied pressure is decreased in a following post-blood-pressure-measurement time period, and wherein the pressure sensor (7) measures the pressure (TP) on the skin at least during the measurement time period, wherein the processor is configured to determine an end measurement time point (32), at or after which the measurement time period is to be stopped, based on the at least one feature determined for at least some of the plurality of pressure pulses (9), and to control the pressure applicator (6, 8) such that, when or after the end measurement time point (32) has been reached, it decreases the applied pressure to start the following post-blood-pressure-measurement time period.

14. A method for determining blood pressure of a subject, wherein the method includes:

- providing a measured pressure signal (TP) of a subject over a time by a pressure signal providing unit, wherein the pressure signal (TP) has been measured by using a measurement device comprising a) a shell configured to encase a part of the subject, through which blood flows, b) a pressure applicator configured to apply, from outside the shell, pressure to the shell and thereby to the encased part of the subject, and c) a pressure sensor configured to measure the pressure signal on the skin of the encased part of the subject, wherein the pressure applicator increases or decreases the applied pressure while the pressure signal is measured, and wherein the measured pressure signal is indicative of blood pulsations and comprises a plurality of pressure pulses (9),
- determining a blood pressure determination curve (TPW M-curve) based on the plurality of pressure pulses (9), wherein the blood pressure determination curve (TPW_M-curve) is indicative for a dependence of the pressure pulses (9) on the applied pressure,
- applying a transformation to the blood pressure determination curve (TPW_M-curve), and
- determining the blood pressure based on a result of the transformation.

15. A computer program for determining blood pressure, the computer program comprising program code means for causing the apparatus for determining blood pressure as defined by any of claims 1 to 13 to carry out the steps of claim 14.

FIG. 1

FIG. 2

FIG. 3

FIG. 4

FIG. 5

FIG. 6

FIG. 7

FIG. 8

FIG. 9

FIG. 10

FIG. 11

FIG. 12

FIG. 13

EP 4 302 687 A1

FIG. 14

Europäisches
Patentamt

European
Patent Office

Office européen
des brevets

# EUROPEAN SEARCH REPORT

Application Number

EP 22 18 9833

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/235996 A1 (CHOI CHANG MOK [KR] ET AL) 5 August 2021 (2021-08-05) * paragraphs [0004], [0051] - [0068]; figures 4,5 * | 1-9,14, 15 | INV. A61B5/022 A61B5/00 |
| X | FOROUZANFAR MOHAMAD ET AL: "Oscillometric Blood Pressure Estimation: Past, Present, and Future", IEEE REVIEWS IN BIOMEDICAL ENGINEERING, vol. 8, 17 August 2015 (2015-08-17), pages 44-63, XP011666732, ISSN: 1937-3333, DOI: 10.1109/RBME.2015.2434215 [retrieved on 2015-08-17] | 1-4,9, 14,15 | |
| A | * II. Background p. 45-47 * * III. OSCILLOMETRIC BP ESTIMATION ALGORITHMS, p. 47-55 * | 5-8 | |
| X | US 2022/096017 A1 (PFEIFFER ULRICH JOACHIM [DE] ET AL) 31 March 2022 (2022-03-31) | 1,9-15 | |
| A | * paragraphs [0054] - [0056], [0071] - [0093], [0101] - [0105] * | 5-8 | **TECHNICAL FIELDS SEARCHED (IPC)** A61B |
| X | DE 10 2017 110770 B3 (UP MED GMBH [DE]) 23 August 2018 (2018-08-23) | 1,9-12, 14,15 | |
| A | * paragraphs [0056], [0070] - [0087] * | 5-8 | |

The present search report has been drawn up for all claims

| Place of search | Date of completion of the search | Examiner |
|---|---|---|
| Berlin | 19 April 2023 | Trachterna, Morten |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 18 9833

This annex lists the patent family members relating to the patent documents cited in the above-mentioned European search report.
The members are as contained in the European Patent Office EDP file on
The European Patent Office is in no way liable for these particulars which are merely given for the purpose of information.

19-04-2023

| Patent document cited in search report | | Publication date | Patent family member(s) | | Publication date |
|---|---|---|---|---|---|
| US 2021235996 | A1 | 05-08-2021 | CN | 113197544 A | 03-08-2021 |
| | | | EP | 3858229 A1 | 04-08-2021 |
| | | | KR | 20210097287 A | 09-08-2021 |
| | | | US | 2021235996 A1 | 05-08-2021 |
| US 2022096017 | A1 | 31-03-2022 | CN | 113573632 A | 29-10-2021 |
| | | | EP | 3952733 A1 | 16-02-2022 |
| | | | JP | 2022518204 A | 14-03-2022 |
| | | | US | 2022096017 A1 | 31-03-2022 |
| | | | WO | 2020148137 A1 | 23-07-2020 |
| DE 102017110770 | B3 | 23-08-2018 | CN | 110913756 A | 24-03-2020 |
| | | | DE | 102017110770 B3 | 23-08-2018 |
| | | | EP | 3624683 A1 | 25-03-2020 |
| | | | JP | 7191093 B2 | 16-12-2022 |
| | | | JP | 2020520292 A | 09-07-2020 |
| | | | WO | 2018210931 A1 | 22-11-2018 |

EPO FORM P0459

For more details about this annex : see Official Journal of the European Patent Office, No. 12/82

**REFERENCES CITED IN THE DESCRIPTION**

**Patent documents cited in the description**

- EP 3430992 A1

- WO 2014121945 A1